# FASCICULE DE BREVET EUROPEEN

(11) **EP 3 294 756 B1**
(45) Date de publication et mention de la délivrance du brevet: **02.12.2020**
(21) Numéro de dépôt: 16729025.3
(22) Date de dépôt: 13.05.2016
(51) Int. Cl.: C07K 14/16, C12N 7/02, C12N 7/04, C12N 15/867, C07K 14/005, C12N 15/86

(54) **PARTICULE RÉTROVIRALE COMPORTANT AU MOINS DEUX ARN NON VIRAUX ENCAPSIDÉS DIFFÉRENTS**
RETROVIRALES PARTIKEL MIT MINDESTENS ZWEI VERSCHIEDENEN KAPSIDIERTEN NICHT-VIRALEN RNAS
RETROVIRAL PARTICLE COMPRISING AT LEAST TWO DIFFERENT ENCAPSIDATED NON-VIRAL RNAS

(30) Priorité: 15.05.2015 FR 1554381; 13.04.2016 FR 1653280
(43) Date de publication de la demande: 21.03.2018
(73) Titulaire: Flash Therapeutics, 31400 Toulouse (FR)
(72) Inventeur: BOUILLE, Pascale, 94300 Vincennes (FR); PAGES, Jean-Christophe, 75019 Paris (FR); GAYON, Régis, 31520 Ramonville Saint-Agne (FR)
(74) Mandataire: Santarelli
(86) Numéro de dépôt international: PCT/FR2016/051152
(87) Numéro de publication internationale: WO 2016/185125

(56) Documents cités:
- WO-A1-2013/148302
- WO-A2-2007/038757
- WO-A2-2007/072056
- M. MARK-DANIELI ET AL: "Single Point Mutations in the Zinc Finger Motifs of the Human Immunodeficiency Virus Type 1 Nucleocapsid Alter RNA Binding Specificities of the Gag Protein and Enhance Packaging and Infectivity", JOURNAL OF VIROLOGY., vol. 79, no. 12, 15 juin 2005 (2005-06-15) , pages 7756-7767, XP055254872, US ISSN: 0022-538X, DOI: 10.1128/JVI.79.12.7756-7767.2005
- TOLGA SUTLU ET AL: "Inhibition of Intracellular Antiviral Defense Mechanisms Augments Lentiviral Transduction of Human Natural Killer Cells: Implications for Gene Therapy", HUMAN GENE THERAPY, vol. 23, no. 10, octobre 2012 (2012-10), pages 1090-1100, XP055255221, US ISSN: 1043-0342, DOI: 10.1089/hum.2012.080 cité dans la demande
- ANNE PREL ET AL: "Highly efficient in vitro and in vivo delivery of functional RNAs using new versatile MS2-chimeric retrovirus-like particles", MOLECULAR THERAPY - METHODS & CLINICAL DEVELOPMENT, vol. 2, 21 octobre 2015 (2015-10-21), page 15039, XP055254802, DOI: 10.1038/mtm.2015.39
- John M Coffin, Stephen H Hughes, Harold E Varmus: "Retroviruses", 1997, Cold Spring Harbor, NY ISBN: 0-87969-571-4 * page 38 - page 40 * * page 124 *

## Description

La présente invention concerne un système rétroviral de transfert d'ARN non viraux dans des cellules cibles et plus particulièrement une particule rétrovirale capable de délivrer de multiples ARN. L'invention concerne également des compositions comportant ces particules rétrovirales, des kits de production de celles-ci, les procédés de fabrication afférents ainsi que les utilisations des particules et des compositions.

Introduire des ARN multiples dans une cellule cible est en enjeu majeur en recherche et développement comme en thérapie génique.

L'utilisation de vecteurs dérivés de virus est devenue une méthode cruciale de transfert de gènes. Les vecteurs viraux se répartissent aujourd'hui en deux catégories principales:
- les vecteurs intégratifs, qui s'intègrent dans le génome receveur, et
- les vecteurs non-intégratifs, qui forment habituellement un élément génétique extra-chromosomique.

Les vecteurs intégratifs tels que les vecteurs gamma-rétroviraux (RV) et vecteurs lentiviraux (LV) sont intégrés de façon stable. Les vecteurs non-intégratifs, tels que les vecteurs adénoviraux (ADV) et les vecteurs adéno-associés (AAV) sont rapidement éliminés des cellules qui se divisent rapidement. Certains facteurs influençant le choix d'un vecteur particulier, comprennent sa capacité d'encapsidation, sa gamme de cellules cibles ou hôtes, son profil d'expression génique, son efficacité de transduction et sa capacité à induire une réponse immunitaire, ce qui est particulièrement problématique si des transductions répétées sont nécessaires.

Certaines applications nécessitent l'utilisation de vecteurs non-intégratifs comme en thérapie génique ou dans de nombreuses applications *in vitro, ex vivo* et *in vivo.* A titre d'exemples, on peut citer :
- l'induction de la reprogrammation de cellules spécialisées en cellules pluripotentes, ainsi que l'induction de la différentiation de cellules souches ou pluripotentes en cellules spécialisées,
- l'expression d'antigènes ou de protéines (toxiques ou non) simultanément dans une cellule cible,
- l'expression de systèmes d'ingénierie du génome comme par exemple la protéine CRE, TALEN ou le système CRISPR, ou de toute autre système nécessitant une expression de protéine ou d'ARN.

Un moyen d'introduire des ARN dans une cellule cible met en œuvre des vecteurs viraux basé sur des virus appartenant à la famille des *Retroviridae* (également désignée sous l'appellation famille des Rétrovirus ou virus à ARN). En effet, durant son cycle de réplication, un virus de la famille des *Retroviridae* possède la capacité de convertir son génome, constitué d'ARN, en ADN double brin qui sera intégré dans le génome de la cellule cible. Des exemples particuliers de cette famille de Rétrovirus sont les gamma-rétrovirus et les lentivirus.

Le cycle réplicatif d'un rétrovirus comporte une première phase de reconnaissance de la cellule cible (ou hôte) via la fixation à un récepteur membranaire. Cette phase de reconnaissance aboutit, après fusion membranaire, à l'entrée du rétrovirus dans la cellule hôte. L'ARN rétroviral est alors copié en ADN double brin par la transcriptase inverse, codée par le rétrovirus, puis intégré au génome de la cellule hôte. Ce génome viral est alors transcrit par la cellule hôte comme tous les autres gènes de la cellule. Ce génome code l'ensemble des protéines et des séquences permettant la fabrication d'autres virus.

Plus particulièrement, trois gènes sont communs à l'ensemble des rétrovirus : *gag, pol* et *env.*

*Gag* est un gène codant pour une polyprotéine, les protéines issues de cette polyprotéine par clivage, étant des protéines de structure impliquées dans l'assemblage des virus lors de la réplication. Ces protéines de structure sont plus spécifiquement la protéine de matrice (MA), la protéine de capside (CA) et la protéine de nucléocapside (NC).

*Pol* est un gène codant pour les enzymes intégrase, transcriptase inverse et protéase.

*Env* est un gène codant pour des glycoprotéines d'enveloppe.

Ces trois gènes permettent donc de copier l'ARN rétroviral en ADN double brin, d'intégrer cet ADN au génome de la cellule hôte puis de générer la structure des rétrovirus néo-synthétisés : protéines d'enveloppe, de capside et de nucléocapside. Toutefois, afin que les rétrovirus néo-synthétisés soient complets, il est nécessaire d'encapsider dans chacune de ces structures deux copies de l'ARN rétroviral. Cette encapsidation de deux copies de l'ARN rétroviral dans la structure s'effectue par la reconnaissance par la protéine de nucléocapside, d'une séquence d'encapsidation appelée Psi (pour « **P**ackaging **S**ignal ») portée par la copie de l'ARN rétroviral.

Lorsqu'un vecteur viral dérivé d'un rétrovirus est utilisé à des fins de thérapie génique, au moins une partie des régions codantes de *gag, pol* et/ou *env* est dissociée entre le système d'encapsidation et le système d'expression de la séquence d'intérêt. Les séquences codantes *gag* et *pol,* par exemple, sont portées par le plasmide d'encapsidation apportant en trans les protéines nécessaires à la production de vecteurs viraux. La séquence d'encapsidation comme la séquence d'intérêt sont portée par des systèmes indépendants, afin de rendre le rétrovirus non réplicatif.

Toutefois, dans certains cas, l'intégration de la séquence d'ARN d'intérêt dans le génome de la cellule hôte de façon aléatoire peut interrompre une phase ouverte de lecture et bloquer l'expression de gènes importants. De plus, pour certaines applications, telles que la reprogrammation de cellules ou la différentiation de cellules souches, il est recommandé que l'expression transitoire d'une séquence d'intérêt soit réalisée de manière transitoire.

La demande WO2007/072056 décrit un système de vectorisation viral comprenant *env, gag,* optionnellement *gag*/*pol* ainsi qu'une séquence d'ARN contenant un signal d'encapsidation hétérologue qui est reconnu par un domaine de liaison à ARN correspondant associé à *gag* ou à *gag*/*pol.* Ce système est décrit dans la demande comme étant non intégratif et permettant une expression transitoire.

Toutefois, l'efficacité de tels systèmes reste limitée. En particulier, pour qu'une cellule cible exprime un ARN d'intérêt véhiculé par ces systèmes, il est généralement nécessaire d'introduire dans la cellule plusieurs copies de cet ARN d'intérêt et par conséquent, d'utiliser de fortes multiplicités d'infection (« multiplicity of infection » ou MOI). La MOI correspond au ratio du nombre de systèmes de vectorisation introduit sur le nombre de cellules à infecter. Une forte MOI permet en effet d'introduire dans les cellules plusieurs copies de l'ARN d'intérêt en permettant qu'une même cellule subisse plusieurs infections. Or, si elle permet d'améliorer le niveau d'expression de l'ARN d'intérêt véhiculé, l'utilisation de fortes MOI, du fait des multiples infections subies par la cellule, engendre également une certaine toxicité.

Il existe donc toujours un besoin pour des systèmes de vectorisation viraux plus efficaces et moins toxiques.

Le travail des inventeurs a permis de réaliser un système de vectorisation capable de délivrer plusieurs ARN d'intérêt dans une même cellule en une seule infection.

La présente divulgation se rapporte à une particule rétrovirale comportant une protéine issue de la polyprotéine Gag, une protéine d'enveloppe, optionnellement une intégrase et au moins deux ARN non viraux encapsidés, les ARN non viraux encapsidés comportant chacun une séquence d'ARN d'intérêt liée à une séquence d'encapsidation, chaque séquence d'encapsidation étant reconnue par un domaine de liaison introduit dans la protéine issue de la polyprotéine Gag et/ou dans l'intégrase.

L'invention est telle que définie dans les revendications.

La présente invention se rapporte à une particule rétrovirale comportant une protéine issue de la polyprotéine Gag, une protéine d'enveloppe, optionnellement une intégrase et au moins deux ARN non viraux différents encapsidés, les ARN non viraux différents encapsidés comportant chacun une séquence d'ARN d'intérêt liée à une séquence d'encapsidation, chaque séquence d'encapsidation étant reconnue par un domaine de liaison introduit dans la protéine issue de la polyprotéine Gag.

La particule rétrovirale selon l'invention permet d'introduire au moins deux ARN non viraux différents, préférentiellement 3, dans une cellule par une seule infection. L'introduction de telles particules dans les cellules peut être effectuée par un procédé *in vitro* ou ex *vivo.*

Par « protéine issue de la polyprotéine Gag », on entend toute protéine résultant du clivage de la polyprotéine Gag. Plus particulièrement, il s'agit d'une protéine de nucléocapside, d'une protéine de matrice (par exemple, dans des particules rétrovirales dérivées du virus murins de type MoMuLV) ou de la protéine p12, spécifique aux gammaretrovirus.

Par « protéine d'enveloppe », on entend toute protéine d'enveloppe, y compris une protéine d'enveloppe de pseudotypage. A titre d'exemple, on peut citer la protéine d'enveloppe Ampho, la protéine d'enveloppe écotrope, la protéine d'enveloppe du virus Moloney de la leucémie murine (MoMuLV), la protéine d'enveloppe du virus de l'Immunodéficience Féline (FIV), la protéine d'enveloppe du virus du sarcome murin d'Harvey (HaMuSV), la protéine d'enveloppe du virus de la tumeur mammaire murine (MuMTV), la protéine d'enveloppe du virus du Sarcôme de Rous (RSV), la protéine d'enveloppe du virus de la rougeole (aussi MV pour *measles* virus), la protéine d'enveloppe du virus de la leucémie du Gibbon (GalV), la protéine du virus endogène félin (RD114) ou la protéine d'enveloppe du virus de la stomatite vésiculaire (VSV-G). Plus particulièrement, la protéine d'enveloppe est la protéine d'enveloppe Ampho, la protéine d'enveloppe écotrope, la protéine d'enveloppe du virus Moloney de la leucémie murine (MoMuLV), la protéine d'enveloppe du virus de l'Immunodéficience Féline (FIV), la protéine d'enveloppe du virus du sarcome murin d'Harvey (HaMuSV), la protéine d'enveloppe du virus de la tumeur mammaire murine (MuMTV), la protéine d'enveloppe du virus du Sarcôme de Rous (RSV), la protéine d'enveloppe du virus de la rougeole (aussi MV pour *measles* virus), la protéine d'enveloppe du virus de la leucémie du Gibbon (GalV) ou la protéine d'enveloppe du virus de la stomatite vésiculaire (VSV-G). La protéine d'enveloppe peut ainsi être modifiée pour cibler certains types cellulaires ou certaines applications (utilisation de récepteurs de surface comme protéine d'enveloppe).

Il est également possible de modifier la protéine d'enveloppe avec un anticorps, un glycolipide et/ou un ligand particulier afin de cibler un récepteur et/ou un type cellulaire particulier.

Préférentiellement, la protéine d'enveloppe est la protéine VSV-G.

Par « intégrase », on entend la protéine enzymatique codée par le gène *pol,* qui permet l'intégration de l'ADN rétroviral dans l'ADN de la cellule infectée par le rétrovirus lors de la réplication dudit rétrovirus.

Par « séquence d'encapsidation », on désigne un motif (séquence et structure tridimensionnelle) ARN reconnu spécifiquement par un domaine de liaison. Préférentiellement, la séquence d'encapsidation est un motif tige-boucle. Plus préférentiellement encore, la séquence d'encapsidation de la particule rétrovirale est le motif tige-boucle de l'ARN du bactériophage MS2 ou du phage PP7 tel que par exemple, celui résultant de la séquence ctagaaaacatgaggatcacccatgtctgcag (SEQ ID N°1) ou (ctagaaggagcagacgatatggcgtcgctccctgcag SEQ ID N°5) respectivement. Le motif tige-boucle et plus particulièrement le motif tige-boucle de l'ARN du bactériophage MS2 ou celui de l'ARN du phage PP7, peut être utilisé seul ou répété plusieurs fois, de préférence de 2 à 25 fois, plus préférentiellement de 2 à 18 fois, par exemple de 6 à 18 fois.

Par « domaine de liaison », on désigne tout ou partie d'une protéine se liant spécifiquement à la séquence d'encapsidation liée à la séquence d'ARN d'intérêt. Plus particulièrement, il s'agit d'une protéine mutante ou non, définie par une structure tridimensionnelle, se liant spécifiquement à la séquence d'encapsidation. Préférentiellement, le domaine de liaison est un domaine hétérologue. Plus préférentiellement, le domaine de liaison est la protéine Coat du bactériophage MS2, du phage PP7 ou du phage Qβ, la protéine nun du prophage HK022, la protéine U1A ou encore la protéine hPum.

Plus préférentiellement, le domaine de liaison est la protéine Coat du bactériophage MS2 ou du phage PP7.

Plus préférentiellement encore, lorsque le domaine de liaison est la protéine Coat du phage PP7, la séquence de celle-ci est déficiente pour l'auto-assemblage, grâce à une délétion des acides aminés 67 à 75 (PCPΔFG) (Chao et al. 2008). Préférentiellement, la séquence de la protéine Coat du phage PP7 est codon-optimisée pour les cellules humaines, c'est-à-dire que les bases de l'ADN sont choisies pour coder pour des acides aminés préférentiellement présents dans l'espèce humaine.

Par « chaque séquence », on entend que les séquences d'encapsidation peuvent être identiques ou différentes, selon que ces séquences d'encapsidation sont reconnues par un domaine de liaison identique ou différent. En effet, la particule rétrovirale selon l'invention peut comporter un ou plusieurs domaines de liaison. Lorsque plusieurs domaines de liaison sont introduits, ceux-ci peuvent l'être dans la polyprotéine Gag.

Le domaine de liaison permet non seulement la reconnaissance de la séquence d'encapsidation mais également l'encapsidation des ARN portant la séquence d'encapsidation dans la particule (ou dans le cas présent, d'un ARN non viral lié à une séquence d'encapsidation).

Par « encapsidation », on entend l'empaquetage d'un ARN dans la capside virale d'une particule virale. On notera que dans la présente invention, l'encapsidation des ARN non viraux s'effectue par la reconnaissance d'un signal d'encapsidation non viral, en particulier autre que Psi.

Par « séquence d'intérêt », on vise une séquence codant pour une fonction présentant un intérêt pour l'utilisateur. Plus particulièrement, la séquence d'intérêt portée par chacun des deux ARN non viraux encapsidés peut être identique ou différente.

A l'aide des particules selon l'invention, il est possible de transférer dans des cellules cibles des ARN non viraux différents.

Les ARN encapsidés étant non viraux, ces ARN ne présentent pas les sites de reconnaissance des protéines codées par le gène *pol.*

En effet, ces ARN non viraux n'entrent pas dans les étapes précoces du cycle réplicatif des rétrovirus, à savoir :
- La copie de l'ARN viral simple brin en ADN double brin par la transcriptase inverse ;
- La maturation de l'ADN viral double brin par reconnaissance des extrémités LTR par l'intégrase et maturation du complexe de pré-intégration cytoplasmique en complexe de pré-intégration nucléaire.

Ces protéines virales (transcriptase inverse, intégrase), codées par le gène pol sont donc optionnelles dans la particule et le gène *pol* peut donc être soit présent, soit délété partiellement ou totalement. Préférentiellement, le gène *pol* est soit présent, soit délété partiellement.

On notera que les particules rétrovirales selon l'invention comportent un matériel génétique à la fois viral et non viral :
- le gène *gag,* qui peut être viral ou chimérique. Plus particulièrement, le gène *gag* est chimérique lorsque le ou les domaine(s) de liaison est/sont introduit(s) dans celui-ci.
- Optionnellement, le gène *pol,* qui peut être viral ou chimérique. Le gène *pol* codant pour plusieurs enzymes, les séquences afférentes à ces enzymes peuvent être délétées totalement ou partiellement, présentes et non fonctionnelles, ou présentes et fonctionnelles. Plus particulièrement, le gène *pol* est chimérique lorsque le ou les domaine(s) de liaison est/sont introduit(s) dans l'intégrase.
- Au moins deux ARN non viraux différents chaque ARN non viral étant porteur d'une séquence d'intérêt et d'une séquence d'encapsidation. Plus particulièrement, ces ARN non viraux sont dépourvus de toute séquence virale.

Plus spécifiquement, les particules rétrovirales selon l'invention permettent d'introduire dans des cellules cibles des ARN capables d'induire :
- Le transfert d'une ou plusieurs séquences d'intérêt codantes endogènes ou exogènes de la cellule cible,
- Le transfert d'un ou plusieurs ARN non codants comme des ARN capables d'induire un effet sur l'expression génétique, par exemple par le biais de shRNA, miRNA, sgRNA, LncRNA ou circRNA.
- Le transfert d'ARN cellulaires, de type ARN messagers ou autres (miRNA...), des réplicons sous génomique de virus à ARN (VHC, etc...) ou de génomes complets de virus à ARN,
- l'expression simultanée de séquences codantes ou non codantes endogènes, exogènes de la cellule cible,
- participer à la modification du génome de la cellule cible par des systèmes d'ingénierie du génome, par exemple le système CRISPR.

Avantageusement, la particule rétrovirale selon l'invention comporte une protéine de nucléocapside, une protéine d'enveloppe, optionnellement une intégrase et au moins deux ARN non viraux différents encapsidés, les ARN non viraux différents encapsidés comportant chacun une séquence d'ARN d'intérêt liée à une séquence d'encapsidation, chaque séquence d'encapsidation étant reconnue par un domaine de liaison introduit dans la protéine de nucléocapside.

Par « protéine de nucléocapside », on entend la protéine de structure NC codée par le gène *gag.* Lorsque le domaine de liaison est introduit dans la protéine de nucléocapside, alors cette protéine est une protéine chimérique, issue d'un gène *gag* chimérique.

Lorsque le domaine de liaison est introduit dans l'intégrase, alors l'intégrase est une protéine chimérique, issue d'un gène *pol* chimérique.

Par « protéine chimérique », on désigne une protéine recombinante comprenant plusieurs séquences protéiques différentes fusionnées.

Selon un premier mode de réalisation, dans la particule rétrovirale selon l'invention, le domaine de liaison est introduit dans la protéine de nucléocapside et les au moins deux ARN non viraux encapsidés se distinguent par leur séquence d'ARN.

Ce premier mode de réalisation permet une expression précoce et transitoire des ARN d'intérêt, sans événement d'intégration associé dans le génome des cellules cibles.

En effet, lors de la mise en contact d'une particule selon ce premier mode de réalisation avec une cellule cible, la membrane de la particule rétrovirale et celle de la cellule cible vont fusionner et permettre la libération du contenu de la particule dans la cellule cible. Les ARN sont alors libérés dans le cytoplasme et la machinerie cellulaire permet de traduire directement ces ARN en protéine(s), c'est-à-dire sans étapes supplémentaires comme la transcription inverse, la translocation dans le noyau ou l'intégration dans le génome de la cellule cible.

Plus particulièrement, les au moins deux ARN non viraux présentent la même séquence d'encapsidation. Dans ce cas, les au moins deux ARN non viraux encapsidés se distinguent par leur séquence d'ARN d'intérêt, c'est-à-dire que les séquences d'ARN d'intérêt comprises dans les deux ARN non viraux encapsidés sont différentes. Par « différentes », on vise des séquences d'intérêt non identiques ou présentant une différence ne résultant pas d'une mutation spontanée ou non intentionnellement choisie par le manipulateur.

Alternativement, les au moins deux ARN non viraux présentent deux séquences d'encapsidation différentes. Ces au moins deux séquences d'encapsidation sont alors reconnues par au moins deux domaines de liaison différents, au moins un de ces domaines étant introduit dans la protéine de nucléocapside. Dans ce cas, les au moins deux ARN non viraux encapsidés peuvent comporter des séquences d'intérêt identiques ou différentes.

Il est possible d'encapsider au moins deux ARN non viraux, préférentiellement trois ARN non viraux.

Selon un mode de réalisation particulier du premier mode de réalisation, un second domaine de liaison est introduit dans la protéine de nucléocapside de la particule rétrovirale selon l'invention.

A titre d'exemple, le second domaine de liaison peut être la protéine « Coat » du bactériophage MS2 lorsque le premier domaine de liaison est la protéine « Coat » du phage PP7 ou le second domaine de liaison peut être la protéine « Coat » du phage PP7 lorsque le premier domaine de liaison est la protéine « Coat » du bactériophage MS2.

Dans ce cas, au moins deux ARN non viraux encapsidés portent des séquences d'encapsidation différentes, chaque séquence d'encapsidation correspondant respectivement au premier et second domaine de liaison introduit dans la protéine de nucléocapside.

Plus particulièrement, lorsque trois ARN non viraux sont encapsidés :
- au moins deux des ARN non viraux encapsidés présentent la même séquence d'encapsidation correspondant au premier domaine de liaison et se distinguent uniquement par leur séquence d'ARN d'intérêt,
- le troisième ARN non viral encapsidé peut porter une séquence d'encapsidation identique ou différente. Lorsque la séquence d'encapsidation est différente, celle-ci peut correspondre à un second domaine de liaison introduit dans la protéine de nucléocapside.

D'autres domaines de liaisons peuvent également être introduits dans la protéine de nucléocapside.

Selon la présente divulgation, outre le ou les domaine(s) de liaison introduit(s) dans la protéine de nucléocapside, il est également possible d'introduire un domaine de liaison dans l'intégrase.

L'intégrase est alors une protéine chimérique, issue d'un gène *pol* chimérique.

Préférentiellement, lorsque le domaine de liaison est introduit dans l'intégrase, la séquence de l'intégrase est mutée au niveau du domaine C-terminal afin d'insérer la séquence du domaine de liaison. Plus préférentiellement encore, la séquence de l'intégrase est mutée au niveau du domaine C-terminal de manière à introduire celle de la protéine « Coat » du bactériophage MS2 ou du phage PP7.

Dans ce cas, les au moins deux ARN non viraux encapsidés peuvent porter des séquences d'encapsidation différentes, chaque séquence d'encapsidation correspondant aux domaines de liaison introduits dans la protéine de nucléocapside et dans l'intégrase respectivement.

Plus particulièrement, lorsque trois ARN non viraux sont encapsidés :
- au moins deux des ARN non viraux encapsidés présentent la même séquence d'encapsidation correspondant au premier domaine de liaison et se distinguent uniquement par leur séquence d'ARN d'intérêt,
- le troisième ARN non viral encapsidé portant une séquence d'encapsidation différente, correspondant à un second domaine de liaison introduit dans l'intégrase.

D'autres domaines de liaisons peuvent également être introduits dans l'intégrase.

Avantageusement, la particule rétrovirale selon l'invention est une particule lentivirale.

Préférentiellement, dans une telle particule lentivirale :
- le domaine de liaison est la séquence de la protéine « Coat » du bactériophage MS2,
- la séquence d'encapsidation des ARN non viraux est une séquence tige-boucle de MS2,
- la protéine de nucléocapside est la protéine de nucléocapside (NC) de HIV appartenant à la polyprotéine Gag, chimérique, la séquence de NC étant mutée au niveau du second doigt de zinc afin d'insérer la séquence de la protéine « Coat » du bactériophage MS2.

Avantageusement :
- La protéine d'enveloppe est la protéine VSV-G codant pour la protéine d'enveloppe du Virus de la stomatite vésiculaire.
- La séquence d'encapsidation comporte de 2 à 25 répétitions de la séquence tige-boucle de MS2, préférentiellement, de 6 à 18 répétitions de la séquence tige-boucle, plus préférentiellement encore de 10 à 14, par exemple 12 répétitions. De préférence, la séquence tige-boucle est la suivante : ctagaaaacatgaggatcacccatgtctgcag (SEQ ID N°1).

Ou, préférentiellement, dans une telle particule lentivirale :
- le domaine de liaison est la séquence de la protéine « Coat » du phage PP7,
- la séquence d'encapsidation des ARN non viraux est une séquence tige-boucle de PP7,
- la protéine de nucléocapside est la protéine de nucléocapside (NC) de HIV appartenant à la polyprotéine Gag, chimérique, la séquence de NC étant mutée au niveau du second doigt de zinc afin d'insérer la séquence de la protéine « Coat » du phage PP7.

Avantageusement :
- La protéine d'enveloppe est la protéine VSV-G codant pour la protéine d'enveloppe du Virus de la stomatite vésiculaire.
- La séquence d'encapsidation comporte de 2 à 25 répétitions de la séquence tige-boucle de PP7, préférentiellement, de 2 à 18 répétitions de la séquence tige-boucle, plus préférentiellement encore de 2 à 12, par exemple 6 répétitions. De préférence, la séquence tige-boucle est la suivante : ctagaaggagcagacgatatggcgtcgctccctgcag (SEQ ID N°5).

Optionnellement, selon la présente divulgation, le second domaine de liaison introduit dans l'intégrase peut être la protéine Coat du phage PP7 si le premier domaine de liaison est la protéine Coat du bactériophage MS2, ou le second domaine de liaison introduit dans l'intégrase peut être la protéine Coat du bactériophage MS2 si le premier domaine de liaison est la protéine Coat du phage PP7.

Selon un second mode de réalisation ne faisant pas partie de la présente invention, la divulgation concerne une particule rétrovirale comportant une protéine issue de la polyprotéine Gag, préférentiellement une protéine de nucléocapside, une protéine d'enveloppe, une intégrase et au moins deux ARN non viraux encapsidés, les ARN non viraux encapsidés comportant chacun une séquence d'ARN d'intérêt liée à une séquence d'encapsidation, chaque séquence d'encapsidation étant reconnue par un domaine de liaison introduit dans l'intégrase et, optionnellement par un domaine de liaison introduit dans une protéine issue de la polyprotéine Gag, préférentiellement une protéine de nucléocapside.

Ce second mode de réalisation permet une expression transitoire des ARN d'intérêt, sans événement d'intégration associé dans le génome des cellules cibles.

Préférentiellement, dans ce second mode de réalisation, la séquence de l'intégrase est mutée au niveau du domaine C-terminal afin d'insérer la séquence du domaine de liaison.

Avantageusement, le domaine de liaison est un domaine hétérologue. Plus particulièrement, le domaine de liaison est la protéine Coat du bactériophage MS2, du phage PP7 ou du phage Qβ, la protéine nun du prophage HK022, la protéine U1A ou encore la protéine hPum.

De préférence, la séquence de l'intégrase est mutée au niveau du domaine C-terminal de manière à introduire celle de la protéine « Coat » du bactériophage MS2 ou du phage PP7.

Les au moins deux ARN non viraux peuvent présenter ou non la même séquence d'encapsidation.

De même, les au moins deux ARN non viraux encapsidés peuvent présenter ou non la même séquence d'ARN d'intérêt.

Préférentiellement, les au moins deux ARN non viraux encapsidés se distinguent par leur séquence d'ARN d'intérêt c'est-à-dire que les séquences d'ARN d'intérêt comprises dans les deux ARN non viraux encapsidés sont différentes.

Plus particulièrement, les au moins deux ARN non viraux présentent la même séquence d'encapsidation, celle-ci étant reconnue par le domaine de liaison introduit dans l'intégrase.

Il est possible d'encapsider au moins deux ARN non viraux, préférentiellement trois ARN non viraux.

Selon un mode de réalisation particulier du second mode de réalisation, un second domaine de liaison est introduit dans l'intégrase de la particule rétrovirale selon la divulgation.

A titre d'exemple, le second domaine de liaison peut être la protéine « Coat » du bactériophage MS2 lorsque le premier domaine de liaison est la protéine « Coat » du phage PP7 ou le second domaine de liaison peut être la protéine « Coat » du phage PP7 lorsque le premier domaine de liaison est la protéine « Coat » du bactériophage MS2.

Dans ce cas, au moins deux ARN non viraux encapsidés portent des séquences d'encapsidations différentes, chaque séquence d'encapsidation correspondant respectivement au premier et second domaine de liaison introduit dans l'intégrase.

Plus particulièrement, lorsque trois ARN non viraux sont encapsidés :
- au moins deux des ARN non viraux encapsidés présentent la même séquence d'encapsidation correspondant au premier domaine de liaison, ces deux ARN non viraux pouvant éventuellement se distinguer par leur séquence d'ARN d'intérêt,
- le troisième ARN non viral encapsidé peut porter une séquence d'encapsidation identique ou différente. Lorsque la séquence d'encapsidation est différente, celle-ci peut correspondre à un second domaine de liaison introduit dans l'intégrase.

D'autres domaines de liaisons peuvent également être introduits dans l'intégrase.

Outre le ou les domaine(s) de liaison introduit(s) dans l'intégrase, il est également possible d'introduire un domaine de liaison dans la protéine de nucléocapside.

La protéine de nucléocapside est alors une protéine chimérique, issue d'un gène *gag* chimérique.

Dans ce cas, les au moins deux ARN non viraux encapsidés peuvent porter des séquences d'encapsidation différentes, chaque séquence d'encapsidation correspondant aux domaines de liaison introduits dans l'intégrase et dans la protéine de nucléocapside respectivement.

Plus particulièrement, lorsque trois ARN non viraux sont encapsidés :
- au moins deux des ARN non viraux encapsidés présentent la même séquence d'encapsidation correspondant au premier domaine de liaison introduit dans l'intégrase, ces ARN non viraux pouvant éventuellement se distinguer par leur séquence d'ARN d'intérêt,
- le troisième ARN non viral encapsidé porte une séquence d'encapsidation différente, correspondant à un second domaine de liaison introduit dans la protéine de nucléocapside.

D'autres domaines de liaisons peuvent également être introduits dans la protéine de nucléocapside.

Avantageusement, la particule rétrovirale selon la divulgation est une particule lentivirale.

Lorsque la particule rétrovirale est une particule lentivirale, il est possible d'exprimer transitoirement des ARN d'intérêt, sans événement d'intégration associé dans le génome des cellules cibles, en particulier des cellules quiescentes.

En effet, en dehors de son rôle dans la réaction d'intégration elle-même, l'intégrase (IN) participe à différentes étapes du cycle réplicatif des rétrovirus comme la morphogénèse de la particule virale, la transcription inverse et l'import nucléaire du complexe de préintégration (PIC).

Plus particulièrement, chez les lentivirus, l'intégrase contient des séquences de localisation nucléaire (NLS) permettant sa localisation dans le noyau grâce au PIC. Par conséquent, lorsque l'encapsidation des ARN non viraux est effectuée par un domaine de liaison porté par une intégrase d'un lentivirus, les ARN non viraux encapsidés seront transportées dans le noyau de la cellule cible. En effet, lors de la mise en contact d'une particule lentivirale selon ce second mode de réalisation avec une cellule cible, la membrane de la particule et celle de la cellule cible vont fusionner et permettre la libération du contenu de la capside dans la cellule cible. Les ARN sont alors pris en charge par l'intégrase qui, via les PIC, va permettre l'import des ARN dans le noyau. Cette prise en charge est particulièrement intéressante pour certaines applications, telles que l'expression dans des cellules quiescentes. Dans le cas de particules rétrovirales, autres que les lentivirus, l'intégrase ne contient pas ces NLS et se trouve donc localisée dans le cytoplasme. Il est toutefois possible de rajouter dans ce type d'intégrase, les séquences NLS afin d'induire une localisation nucléaire de l'intégrase, et donc des ARN pris en charge par cette intégrase.

Cette prise en charge est également particulièrement utile pour un système CRISPR, qui fait appel à des guides ARN qui viennent s'hybrider spécifiquement au génome de la cellule cible. Une fois hybridés, ces guides ARN guident une endonucléase (Cas9), qui va permettre la modification d'un locus spécifique du génome de la cellule cible.

Plus particulièrement, dans une telle particule lentivirale :
- le domaine de liaison est la protéine « Coat » du bactériophage MS2,
- la séquence d'encapsidation des ARN non viraux est une séquence tige-boucle de MS2,
- l'intégrase est une protéine enzymatique chimérique dont la séquence est mutée au niveau du domaine C-terminal afin d'insérer la séquence de la protéine « Coat » du bactériophage MS2.

Ou, plus particulièrement, dans une telle particule lentivirale :
- le domaine de liaison est la protéine « Coat » du phage PP7,
- la séquence d'encapsidation des ARN non viraux est une séquence tige-boucle de PP7,
- l'intégrase est une protéine enzymatique chimérique dont la séquence est mutée au niveau du domaine C-terminal afin d'insérer la séquence de la protéine « Coat » du phage PP7.

Optionnellement, le second domaine de liaison introduit dans la nucléocapside peut être la protéine Coat du phage PP7 si le premier domaine de liaison est la protéine Coat du bactériophage MS2 ou le second domaine de liaison introduit dans l'intégrase peut être la protéine Coat du bactériophage MS2 si le premier domaine de liaison est la protéine Coat du phage PP7.

En effet, l'intégrase (IN) est composée de 3 domaines fonctionnels distincts, chacun indispensable pour assurer une réaction d'intégration complète. Le domaine N-terminal contient un motif de type doigt de zinc qui stabilise la structure repliée de l'IN et augmente l'activité catalytique de l'enzyme. Le domaine central de l'IN contient le motif d'acides aminés DDE auquel est attribué l'activité catalytique de l'enzyme. Ce domaine central est aussi impliqué dans la reconnaissance de la séquence nucléotidique conservée à chaque extrémité de l'ADN rétroviral. Le domaine C-terminal est le moins conservé parmi la famille des rétrovirus. Il possède l'activité de liaison à l'ADN et est indispensable pour les réactions de maturation des extrémités 3' de transfert de brin. En dehors de son rôle dans la réaction d'intégration elle-même, IN participe à différentes étapes du cycle réplicatif des rétrovirus comme la morphogénèse de la particule virale, la transcription inverse et l'import nucléaire du complexe de préintégration.

Comme décrit par Petit et al. (1999; J. Virol. P5079-5088), l'insertion d'une séquence exogène en C-terminal de l'IN ne perturbe pas les étapes de production et de transduction de cellules cibles alors qu'une même insertion en N-terminal ne permet pas la détection d'un événement de transduction.

La particule rétrovirale a donc été modifiée pour contenir la protéine « Coat » du bacteriophage MS2 en fusion avec la protéine de l'intégrase (voir Figure I) ou la protéine « Coat » du phage PP7 (voir Figure XXXVII). Le plasmide d'encapsidation p8.74, porteur du gène *pol* codant pour la protéine de l'intégrase, est modifié afin d'insérer la séquence codant la protéine Coat en C-terminal de l'intégrase par PCR d'assemblage. Le plasmide p8.74 est linéarisé par PCR puis la séquence Coat, préalablement amplifiée par PCR, est clonée au niveau C-terminal de l'intégrase, soit directement bout-à-bout soit avec l'ajout un linker.

Avantageusement :
- La protéine d'enveloppe est la protéine VSV-G codant pour la protéine d'enveloppe du Virus de la stomatite vésiculaire.
- La séquence d'encapsidation comporte de 2 à 25 répétitions de la séquence tige-boucle de MS2 et/ou de PP7, selon le domaine de liaison introduit, préférentiellement, de 2 à 18 répétitions, plus préférentiellement de 2 à 18 répétitions, tel que de 6 à 18 répétitions de la séquence tige-boucle, plus préférentiellement encore pour la séquence tige-boucle de MS2, de 10 à 14, par exemple 12 répétitions.

- Préférentiellement, la séquence tige-boucle est la suivante : ctagaaaacatgaggatcacccatgtctgcag (SEQ ID N°1) lorsque le domaine de liaison est la protéine Coat de MS2 et/ou la séquence tige-boucle est la suivante : ctagaaggagcagacgatatggcgtcgctccctgcag (SEQ ID N°5) lorsque le domaine de liaison est la protéine Coat de PP7.

Plusieurs exemples de particule lentivirale selon l'invention sont décrits de manière plus détaillée dans les exemples qui suivent :
- une particule MS2RLP ou MS2 (NC)-RLP 12X est une particule lentivirale formée par encapsidation d'ARNs portant le motif tige boucle du bactériophage MS2, répété 12 fois, par l'insertion de la protéine Coat du bactériophage MS2 dans la nucléocapside,
- une particule MS2 (NC)-RLP 2X est une particule lentivirale formée par encapsidation d'ARNs portant le motif tige boucle du bactériophage MS2, répété 2 fois, par l'insertion de la protéine Coat du bactériophage MS2 dans la nucléocapside,
- une particule MS2 (NC)-RLP 6X est une particule lentivirale formée par encapsidation d'ARNs portant le motif tige boucle du bactériophage MS2, répété 6 fois, par l'insertion de la protéine Coat du bactériophage MS2 dans la nucléocapside,
- une particule PP7 (NC)-RLP 2X est une particule lentivirale formée par encapsidation d'ARNs portant le motif tige boucle du phage PP7, répété 2 fois, par l'insertion de la protéine Coat du phage PP7 dans la nucléocapside,
- une particule PP7 (NC)-RLP 6X est une particule lentivirale formée par encapsidation d'ARNs portant le motif tige boucle du phage PP7, répété 6 fois, par l'insertion de la protéine Coat du phage PP7 dans la nucléocapside,
- une particule PP7RLP ou PP7 (NC)-RLP 12X est une particule lentivirale formée par encapsidation d'ARNs portant le motif tige boucle du phage PP7, répété 12 fois, par l'insertion de la protéine Coat du phage PP7 dans la nucléocapside.

Préférentiellement, la particule lentivirale formée par encapsidation d'ARNs porte un motif tige boucle du bactériophage MS2 ou du phage PP7, répété de 2 à 25 fois, préférentiellement encore 2, 6 ou 12 fois.

Plus préférentiellement encore, la particule selon l'invention est choisie parmi MS2 (NC)-RLP 12X, PP7 (NC)-RLP 6X et PP7 (NC)-RLP 2X.

L'invention concerne également des compositions comportant des particules selon l'invention.

Plus particulièrement, les compositions selon l'invention sont des compositions concentrées. Avantageusement, les compositions sont également purifiées. Ces compositions peuvent être concentrées et purifiées selon le procédé décrit dans la demande WO2013/014537.

Typiquement, les compositions selon l'invention comportent moins de 30 % de contaminants ADN et moins de 45% de contaminants protéiques par rapport au surnageant brut. Plus particulièrement, les compositions selon l'invention comportent moins de 30 % de contaminants ADN et moins de 2% de contaminants protéiques par rapport au surnageant brut.

Par « surnageant brut », on vise le surnageant de culture(s) cellulaire(s), comportant des particules rétrovirales selon l'invention, après clarification. Une telle étape de clarification est plus particulièrement décrite ci-après dans les procédés de fabrication des particules selon l'invention. Lorsque la récupération du surnageant est effectué en plusieurs fois, le surnageant brut correspond alors à l'ensemble des surnageants collectés, réunis (ou « poolés ») puis clarifié.

Optionnellement, les compositions selon l'invention comportent moins de 1 % de contaminants ADN et moins de 1% de contaminants protéiques par rapport au surnageant brut.

L'invention se rapporte aussi à des kits de production de particules rétrovirales selon l'invention et aux procédés de fabrication de ces kits, tels que définis dans les revendications.

Plus particulièrement, la présente divulgation concerne un kit de production de particules selon l'invention, comportant :
(i) un plasmide d'expression comportant au moins une séquence d'intérêt, pour laquelle en amont ou en aval de cette séquence est insérée une séquence d'encapsidation,
(ii) un plasmide d'encapsidation codant pour une protéine issue de la polyprotéine Gag et/ou une intégrase, chimérique, comportant un domaine de liaison permettant de reconnaître une séquence d'encapsidation, et,
(iii) un plasmide d'enveloppe codant pour une protéine d'enveloppe.

Un tel kit peut également comporter une notice d'utilisation des plasmides contenus dans le kit.

Plus spécifiquement, lorsque le kit vise à produire des particules rétrovirales selon le premier mode de réalisation, ce kit selon l'invention comporte :
(i) un plasmide d'expression codant au moins deux séquences d'ARN non virales différentes, chaque séquence d'ARN comportant une séquence d'intérêt pour laquelle en amont ou en aval de cette séquence d'intérêt est insérée une séquence d'encapsidation, ou alternativement un premier et un second plasmide d'expression codant chacun une séquence d'ARN non viral différente comportant une séquence d'intérêt et en amont ou en aval de ladite séquence d'intérêt est insérée une séquence d'encapsidation,
(ii) un plasmide d'encapsidation codant pour une protéine de nucléocapside chimérique comportant un domaine de liaison permettant de reconnaître la séquence d'encapsidation, et,
(iii) un plasmide d'enveloppe codant pour une protéine d'enveloppe.

Les au moins deux séquences d'ARN non virales sont différentes parce que les séquences d'intérêt sont différentes et/ou les séquences d'encapsidation sont différentes.

Préférentiellement, le kit produisant des particules selon le premier mode de réalisation comporte :
(i) un plasmide d'expression comportant au moins deux séquences d'intérêt, pour lesquelles en amont ou en aval de chacune de ces séquences est insérée une séquence d'encapsidation, ou alternativement un premier et un second plasmide d'expression comportant chacun une séquence d'intérêt en amont ou en aval de laquelle est insérée une séquence d'encapsidation,
   les séquences d'intérêt étant différentes et les séquences d'encapsidation étant identiques,
(ii) un plasmide d'encapsidation codant pour une protéine de nucléocapside chimérique comportant un domaine de liaison permettant de reconnaître la séquence d'encapsidation, et,
(iii) un plasmide d'enveloppe codant pour une protéine d'enveloppe. Avantageusement, le kit produit des particules lentivirales.

Préférentiellement :
- Dans le plasmide d'expression, la séquence d'encapsidation comporte de 2 à 25 répétitions de la séquence tige-boucle de MS2, préférentiellement de 6 à 18 répétitions de la séquence tige-boucle, plus préférentiellement encore de 10 à 14, par exemple 12 répétitions. Avantageusement, la séquence tige-boucle est la suivante : ctagaaaacatgaggatcacccatgtctgcag (SEQ ID N°1).
- Dans le plasmide d'encapsidation, la protéine de nucléocapside est la protéine de nucléocapside (NC) de HIV appartenant à la polyprotéine Gag, laquelle NC est mutée au niveau du second doigt de zinc afin d'insérer la séquence de la protéine « Coat » du bactériophage MS2.
- Dans le plasmide d'enveloppe, la protéine d'enveloppe est la protéine VSV-G codant pour la protéine d'enveloppe du Virus de la stomatite vésiculaire.

Ou, préférentiellement :
- Dans le plasmide d'expression, la séquence d'encapsidation comporte de 2 à 25 répétitions de la séquence tige-boucle de PP7, préférentiellement, de 2 à 18 répétitions de la séquence tige-boucle, plus préférentiellement encore de 2 à 12, par exemple 6 répétitions. Avantageusement, la séquence tige-boucle est la suivante : ctagaaggagcagacgatatggcgtcgctccctgcag (SEQ ID N°5).
- Dans le plasmide d'encapsidation, la protéine de nucléocapside est la protéine de nucléocapside (NC) de HIV appartenant à la polyprotéine Gag, laquelle NC est mutée au niveau du second doigt de zinc afin d'insérer la séquence de la protéine « Coat » du phage PP7.
- Dans le plasmide d'enveloppe, la protéine d'enveloppe est la protéine VSV-G codant pour la protéine d'enveloppe du Virus de la stomatite vésiculaire.

Optionnellement, le kit comporte un second plasmide d'encapsidation codant pour une intégrase chimérique comportant un domaine de liaison permettant de reconnaître une séquence d'encapsidation. Le second domaine de liaison peut être identique ou différent du domaine de liaison de la protéine de nucléocapside chimérique.

A titre d'exemple de domaines de liaison différents :
- le domaine de liaison introduit dans la nucléocapside peut être la protéine Coat de MS2 et le domaine de liaison introduit dans l'intégrase peut être la protéine Coat de PP7, ou
- le domaine de liaison introduit dans la nucléocapside peut être la protéine Coat de PP7 et le domaine de liaison introduit dans l'intégrase peut être la protéine Coat de MS2.

Plusieurs domaines de liaison peuvent être introduits dans chacune des protéines chimériques.

Alternativement, lorsque le kit vise à produire des particules selon le second mode de réalisation, ne faisant pas partie de l'invention, ce kit comporte :
(i) un plasmide d'expression comportant au moins une séquence d'intérêt, pour laquelle en amont ou en aval de cette séquence est insérée une séquence d'encapsidation,
(ii) un plasmide d'encapsidation codant pour une intégrase chimérique comportant un domaine de liaison permettant de reconnaître la séquence d'encapsidation, et,
(iii) un plasmide d'enveloppe codant pour une protéine d'enveloppe.

Des procédés de fabrication des kits selon l'invention sont également proposés. Typiquement, un procédé de fabrication d'un kit selon la divulgation comporte :
(i) la préparation d'un plasmide d'expression comportant au moins une séquence d'intérêt, pour laquelle en amont ou en aval de cette séquence est insérée une séquence d'encapsidation
(ii) la préparation d'un plasmide d'encapsidation codant pour une protéine issue de la polyprotéine Gag et/ou une intégrase, chimérique, comportant un domaine de liaison permettant de reconnaître une séquence d'encapsidation, et
   la préparation d'un plasmide d'enveloppe codant pour une protéine d'enveloppe.

Plus spécifiquement, lorsque le procédé concerne un kit qui vise à produire des particules rétrovirales selon le premier mode de réalisation, ce procédé selon l'invention comporte :
(i) la préparation d'un plasmide d'expression codant au moins deux séquences d'ARN non virales différentes, chaque séquence d'ARN comportant une séquence d'intérêt pour laquelle en amont ou en aval de cette séquence d'intérêt est insérée une séquence d'encapsidation, ou alternativement un premier et un second plasmide d'expression codant chacun une séquence d'ARN non viral différente comportant une séquence d'intérêt et dans laquelle en amont ou en aval de la séquence d'intérêt est insérée une séquence d'encapsidation,
(ii) la préparation d'un plasmide d'encapsidation codant pour une protéine de nucléocapside chimérique comportant un domaine de liaison permettant de reconnaître la séquence d'encapsidation, et,
(iii) la préparation d'un plasmide d'enveloppe codant pour une protéine d'enveloppe.

Les au moins deux séquences d'ARN non virales sont différentes parce que les séquences d'intérêt sont différentes et/ou les séquences d'encapsidation sont différentes.

Préférentiellement, ce procédé comporte :
(i) la préparation d'un plasmide d'expression comportant au moins deux séquences d'intérêt, pour lesquelles en amont ou en aval de chacune de ces séquences est insérée une séquence d'encapsidation, ou alternativement d'un premier et d'un second plasmide d'expression comportant chacun une séquence d'intérêt en amont ou en aval de laquelle est insérée une séquence d'encapsidation,
   les séquences d'intérêt étant différentes et les séquences d'encapsidation étant identiques,
(ii) la préparation d'un plasmide d'encapsidation codant pour une protéine de nucléocapside chimérique comportant un domaine de liaison permettant de reconnaître la séquence d'encapsidation, et
(iii) la préparation d'un plasmide d'enveloppe codant pour une protéine d'enveloppe.

Alternativement, lorsque le procédé concerne un kit qui vise à produire des particules selon le second mode de réalisation, ce procédé ne faisant pas partie de l'invention, comporte :
(i) la préparation d'un plasmide d'expression comportant au moins une séquence d'intérêt, pour laquelle en amont ou en aval de cette séquence est insérée une séquence d'encapsidation,
(ii) la préparation d'un plasmide d'encapsidation codant pour une intégrase chimérique comportant un domaine de liaison permettant de reconnaître la séquence d'encapsidation, et,
(iii) la préparation d'un plasmide d'enveloppe codant pour une protéine d'enveloppe.

L'invention se rapporte également à des procédés de fabrication des particules rétrovirales selon l'invention.

Un procédé selon la présente divulgation, comporte une étape de co-transfection de cellules avec :
(i) un plasmide d'expression comportant au moins une séquence d'intérêt, pour laquelle en amont ou en aval de cette séquence est insérée une séquence d'encapsidation,
(ii) un plasmide d'encapsidation codant pour une protéine issue de la polyprotéine Gag et/ou une intégrase chimérique comportant un domaine de liaison permettant de reconnaître une séquence d'encapsidation, et,
(iii) un plasmide d'enveloppe codant pour une protéine d'enveloppe.
et la récupération du surnageant des cellules transfectées comportant les particules.

Les cellules mises en œuvre dans les procédés de fabrication de particules selon l'invention sont des cellules productrices, c'est-à-dire des cellules, qui une fois transfectées avec les plasmides portant le matériel génétique nécessaire à la formation des particules rétrovirales, permettent la formation desdites particules. A titre d'exemple de cellules productrices, on peut citer HEK293T.

Par « étape de co-transfection », on entend une étape de transfection lors de laquelle la transfection est effectuée par mise en contact des cellules productrices avec l'ensemble des plasmides du procédé de fabrication des particules.

Plus spécifiquement, lorsque le procédé de fabrication selon l'invention vise à produire des particules rétrovirales selon le premier mode de réalisation, il comporte une étape de co-transfection de cellules avec :
(i) un plasmide d'expression codant au moins deux séquences d'ARN non virales différentes, chaque séquence d'ARN comportant une séquence d'intérêt pour laquelle en amont ou en aval de cette séquence d'intérêt est insérée une séquence d'encapsidation, ou alternativement un premier et un second plasmide d'expression codant chacun une séquence d'ARN non viral différente comportant une séquence d'intérêt et dans laquelle en amont ou en aval de la séquence d'intérêt est insérée une séquence d'encapsidation,
(ii) un plasmide d'encapsidation codant pour une protéine de nucléocapside chimérique comportant un domaine de liaison permettant de reconnaître la séquence d'encapsidation, et,
(iii) un plasmide d'enveloppe codant pour une protéine d'enveloppe,
et la récupération du surnageant des cellules transfectées comportant les particules.

Les au moins deux séquences d'ARN non virales sont différentes parce que les séquences d'intérêt sont différentes et/ou les séquences d'encapsidation sont différentes.

Préférentiellement, ce procédé de fabrication de particules comporte une étape de co-transfection de cellules avec :
(i) un plasmide d'expression comportant au moins deux séquences d'intérêt, pour lesquelles en amont ou en aval de chacune de ces séquences est insérée une séquence d'encapsidation, ou alternativement un premier et un second plasmide d'expression comportant chacun une séquence d'intérêt en amont ou en aval de laquelle est insérée une séquence d'encapsidation,
   les séquences d'intérêt étant différentes et les séquences d'encapsidation étant identiques,
(ii) un plasmide d'encapsidation codant pour une protéine de nucléocapside chimérique comportant un domaine de liaison permettant de reconnaître la séquence d'encapsidation, et,
(iii) un plasmide d'enveloppe codant pour une protéine d'enveloppe,
et la récupération du surnageant des cellules transfectées comportant les particules.

Alternativement, lorsque le procédé de fabrication vise à produire des particules selon le second mode de réalisation, ce procédé ne faisant pas partie de l'invention, comporte une étape de co-transfection de cellules avec :
(i) un plasmide d'expression comportant au moins une séquence d'intérêt, pour laquelle en amont ou en aval de cette séquence est insérée une séquence d'encapsidation,
(ii) un plasmide d'encapsidation codant pour une intégrase chimérique comportant un domaine de liaison permettant de reconnaître la séquence d'encapsidation, et,
(iii) un plasmide d'enveloppe codant pour une protéine d'enveloppe,
et la récupération du surnageant des cellules transfectées comportant les particules.

Préférentiellement, tous les procédés de fabrication de particules rétrovirales selon l'invention sont réalisés conformément aux procédés décrits dans la demande WO2013/014537.

Plus particulièrement, ces procédés de fabrication de particules rétrovirales sont réalisés sur des cellules productrices cultivées en milieu sans sérum et aucune induction au sodium butyrate n'est effectuée.

Avantageusement, le surnageant est collecté en plusieurs fois, par exemple entre 3 et 6 fois, à des intervalles de temps spécifiques, tels qu'à des intervalles de temps de l'ordre de la demi vie des particules rétrovirales. Typiquement, la récupération du surnageant est effectuée en 4 à 5 fois, à des intervalles de temps de l'ordre de 6 à 18h, préférentiellement de 8 à 16h, tels que 8h, 12h et/ou 16h. Préférentiellement, cette collecte est effectuée après changement du milieu de culture des cellules, ce changement étant effectué préférentiellement à 24h post-transfection.

Les procédés de fabrication de particules rétrovirales selon l'invention comportent également une étape dans laquelle le surnageant est clarifié.

Préférentiellement, la clarification du surnageant est effectuée par centrifugation.

Plus préférentiellement encore, ces procédés de fabrication de particules rétrovirales selon l'invention comportent en outre une étape dans laquelle le surnageant est concentré et/ou purifié.

De préférence, la concentration et purification est effectuée par ultrafiltration frontale sur des unités de centrifugation.

Avantageusement, le surnageant subit une ou plusieurs étapes supplémentaires de purification. Ces étapes de purification sont effectuées de préférence par ultrafiltration tangentielle et/ou diafiltration. Plus préférentiellement encore, le surnageant subit une étape d'ultrafiltration tangentielle suivie d'une étape de diafiltration.

L'ultrafiltration tangentielle est avantageusement effectuée sur des cartouches de fibres creuses en polysulfone.

Optionnellement, la composition peut ensuite subir une étape de chromatographie par échange d'ions, en particulier une chromatographie par échange d'anions. L'éluât issu de cette étape de chromatographie est ensuite récupéré puis concentré à nouveau par ultrafiltration frontale sur des unités centrales de centrifugation. Une composition résultant d'un tel procédé comporte moins de 1 % de contaminants ADN et moins de 1% de contaminants protéiques par rapport au surnageant brut.

La présente divulgation concerne également des compositions susceptibles d'être obtenues par l'un quelconque des procédés de fabrication de particules selon l'invention.

Typiquement, ces compositions comportent moins de 30 % de contaminants ADN et moins de 45% de contaminants protéiques par rapport au surnageant brut. Plus particulièrement, les compositions selon la divulgation comportent moins de 30 % de contaminants ADN et moins de 2% de contaminants protéiques par rapport au surnageant brut.

Optionnellement, les compositions selon la divulgation comportent moins de 1 % de contaminants ADN et moins de 1% de contaminants protéiques par rapport au surnageant brut.

Enfin, l'invention concerne l'utilisation d'une particule selon l'invention, ou d'une composition selon l'invention pour transduire des cellules.

L'utilisation des particules et des compositions selon l'invention est particulièrement avantageuse pour transduire des cellules primaires et des lignées immortalisées, afin de les modifier de façon transitoire. Les cellules peuvent être des cellules de mammifères ou d'autres eucaryotes. En particulier, la transduction de ces cellules peut être effectuée *in vitro* ou *ex vivo.*

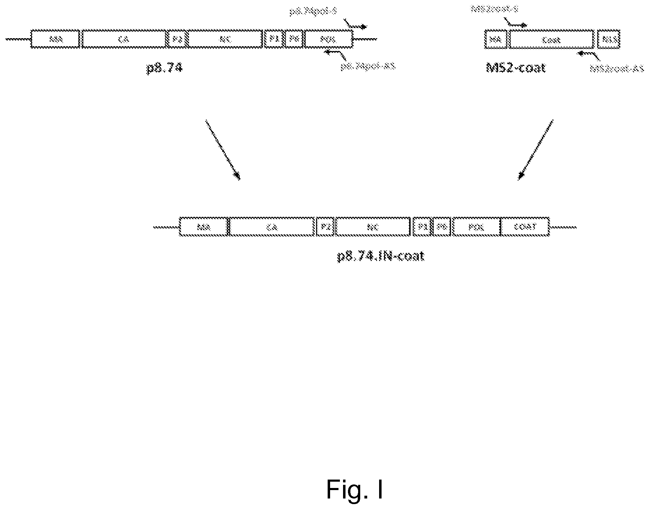

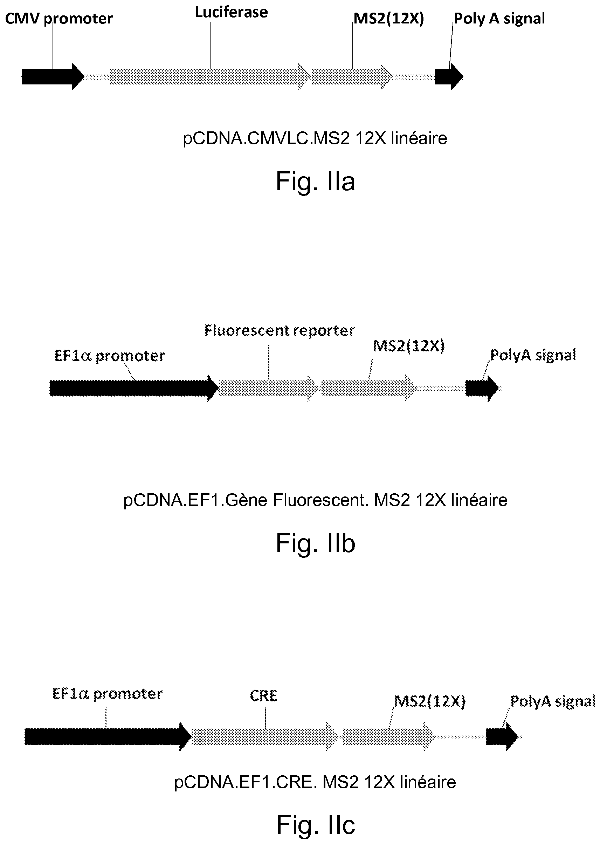

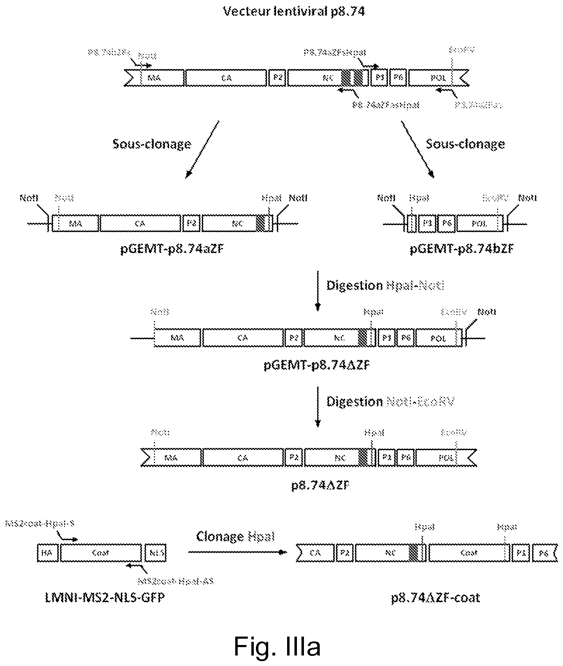

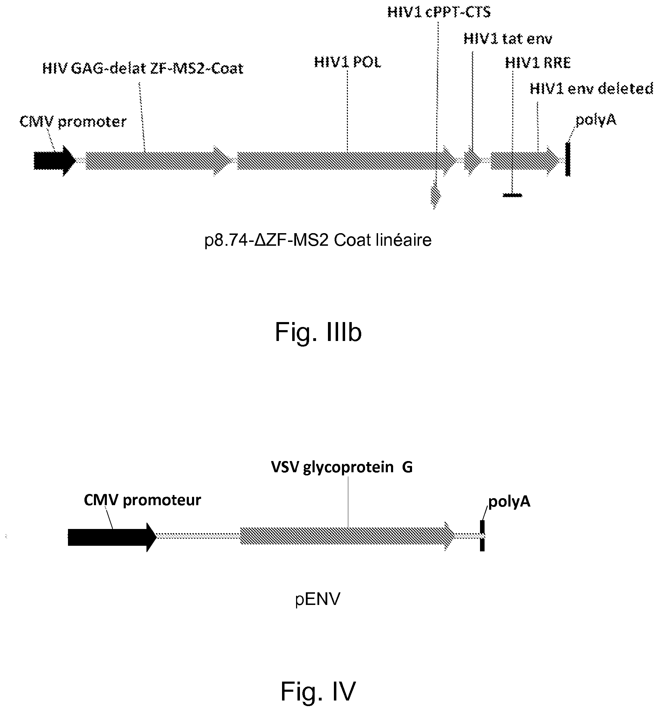

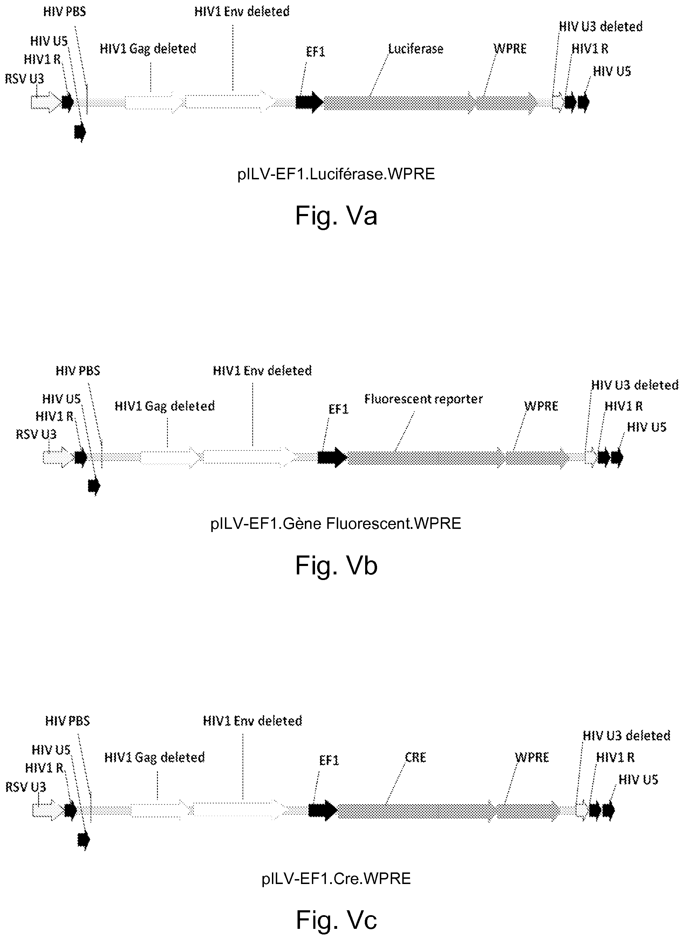

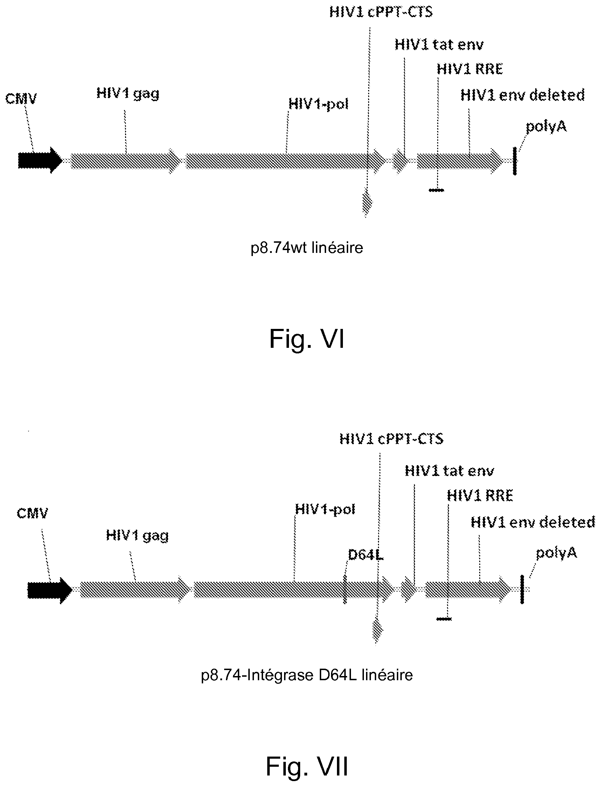

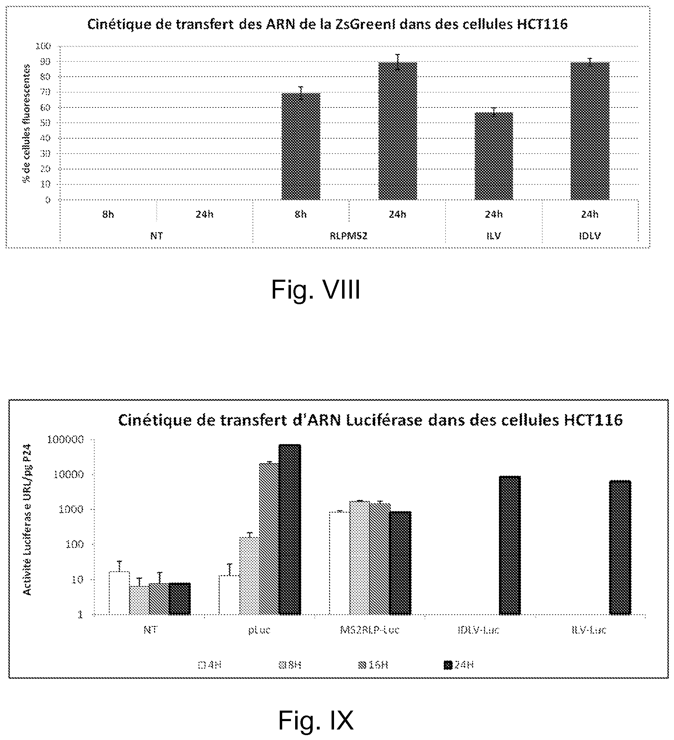

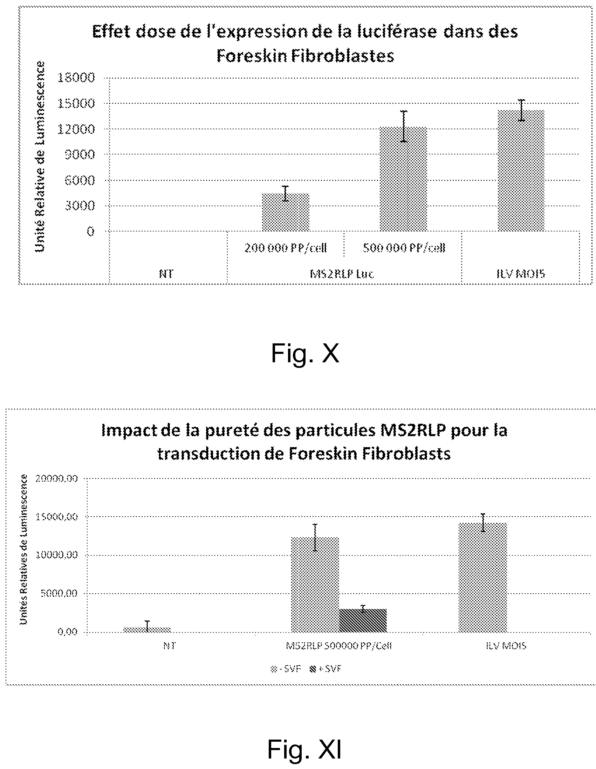

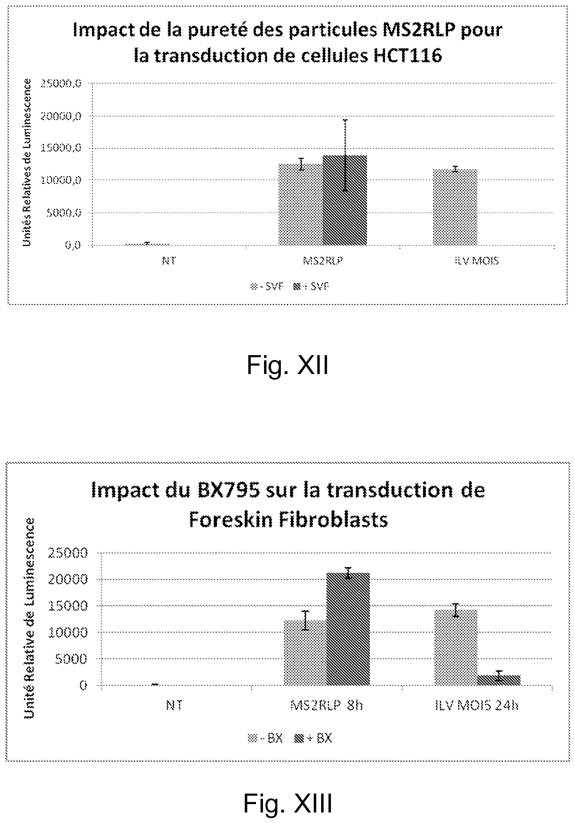

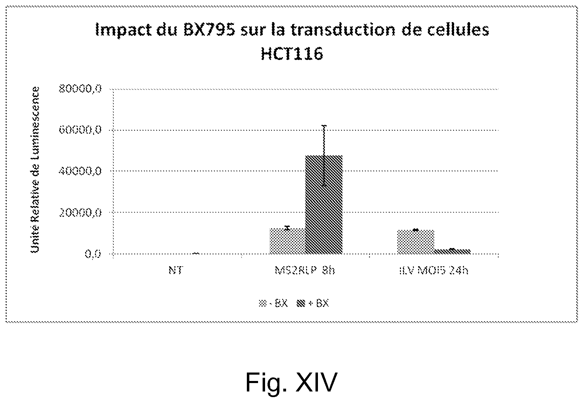

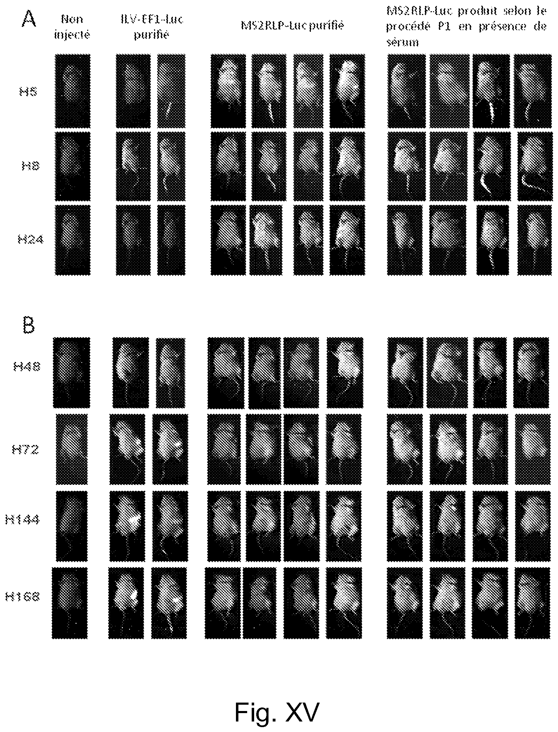

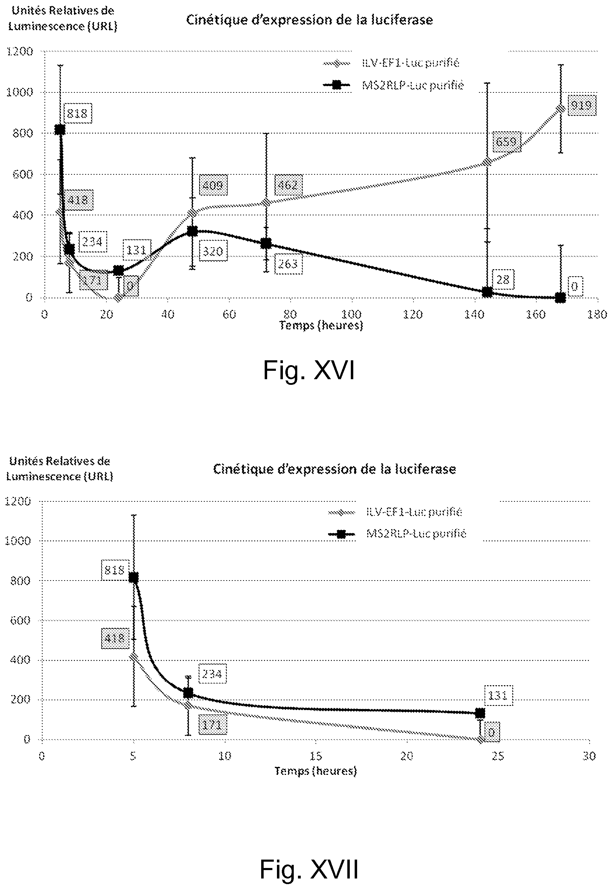

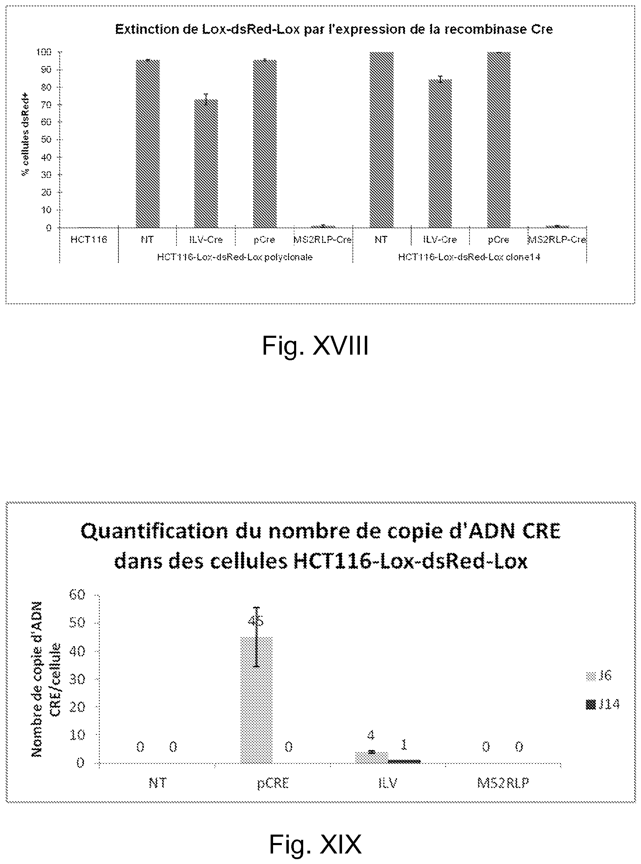

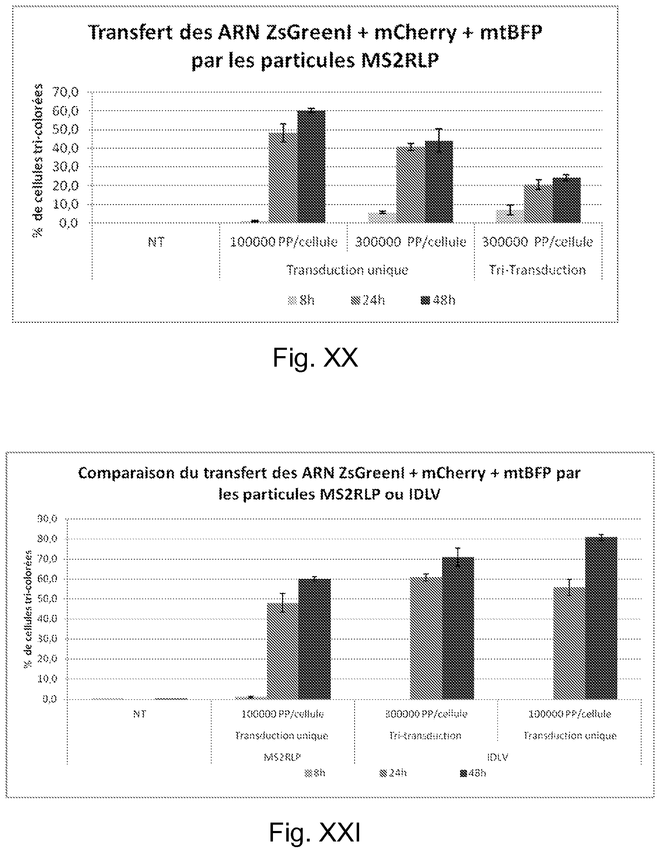

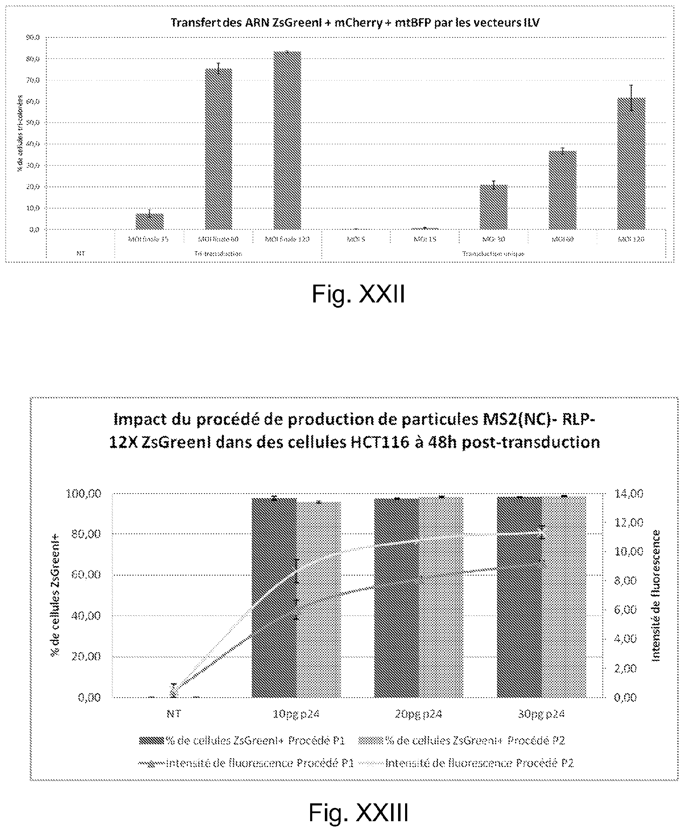

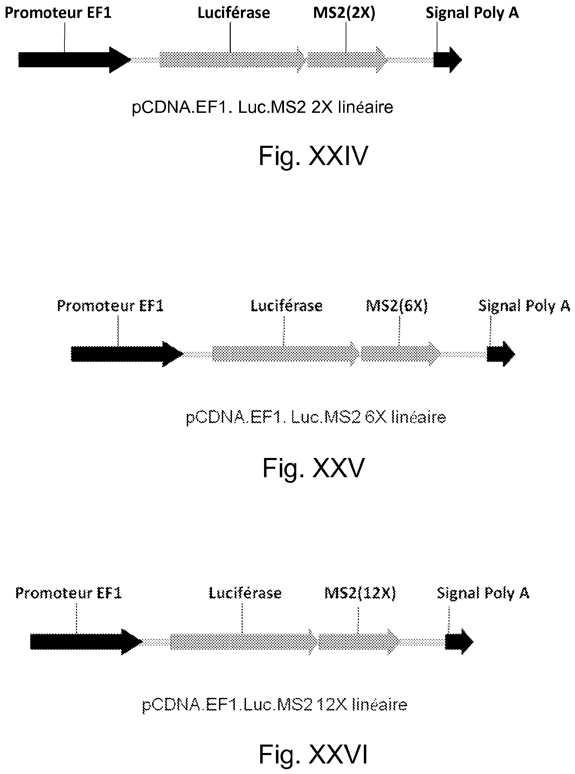

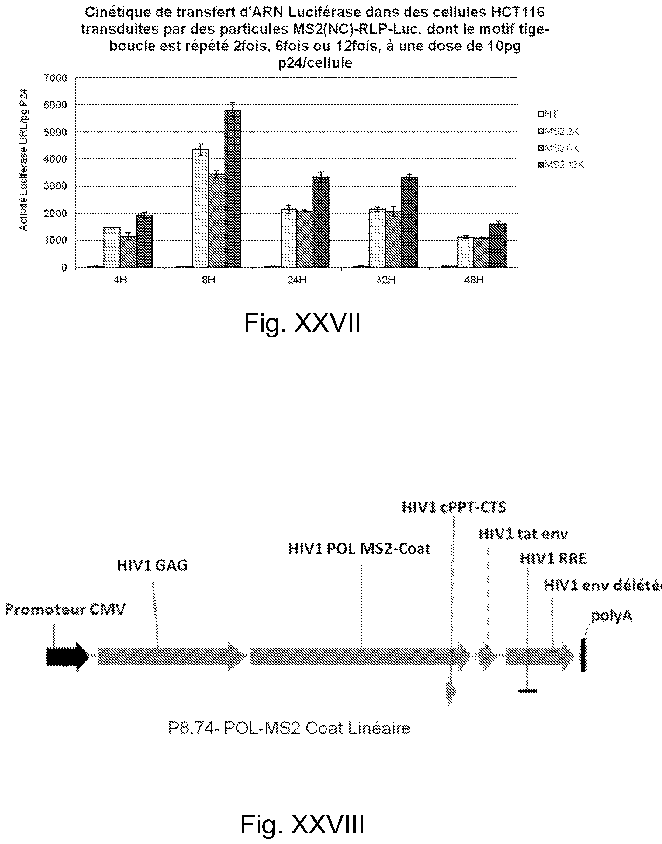

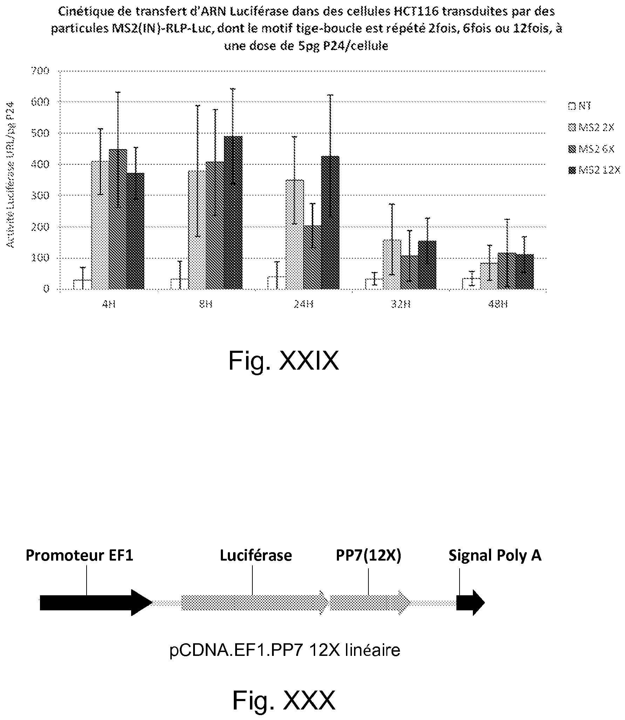

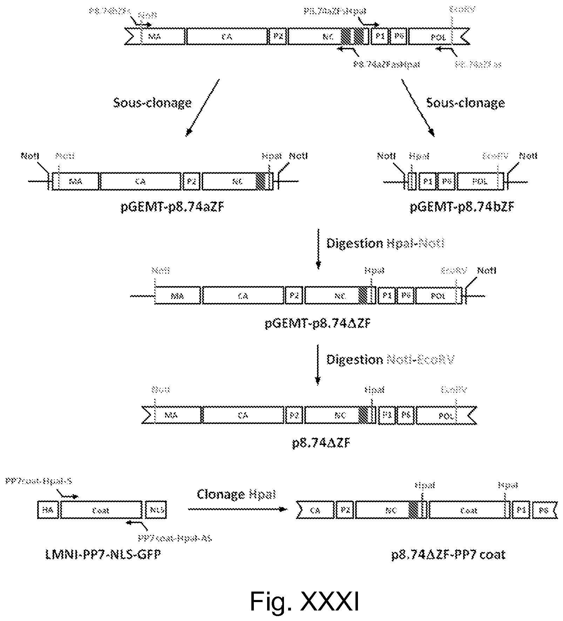

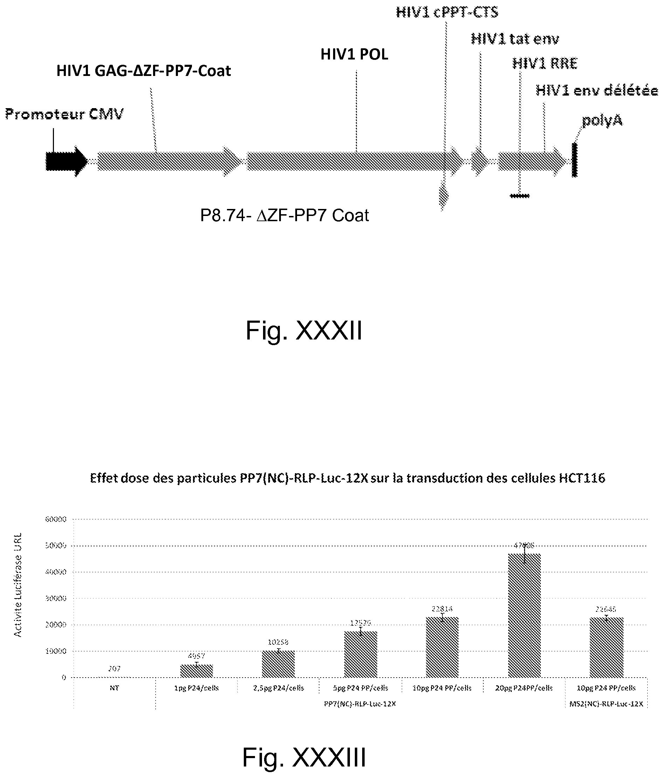

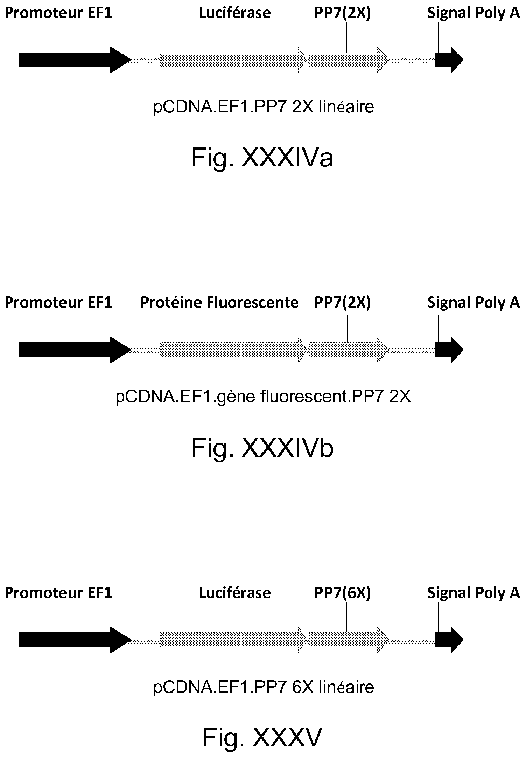

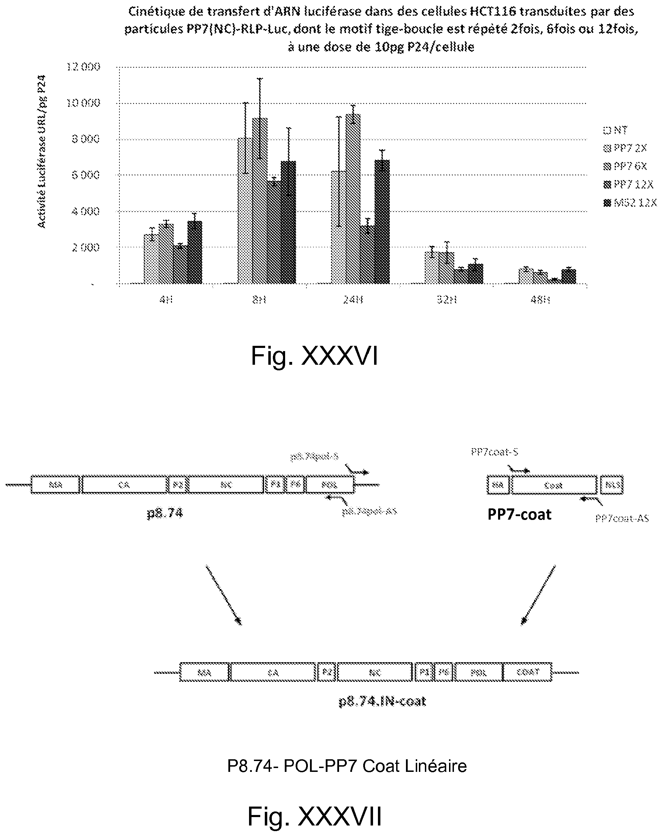

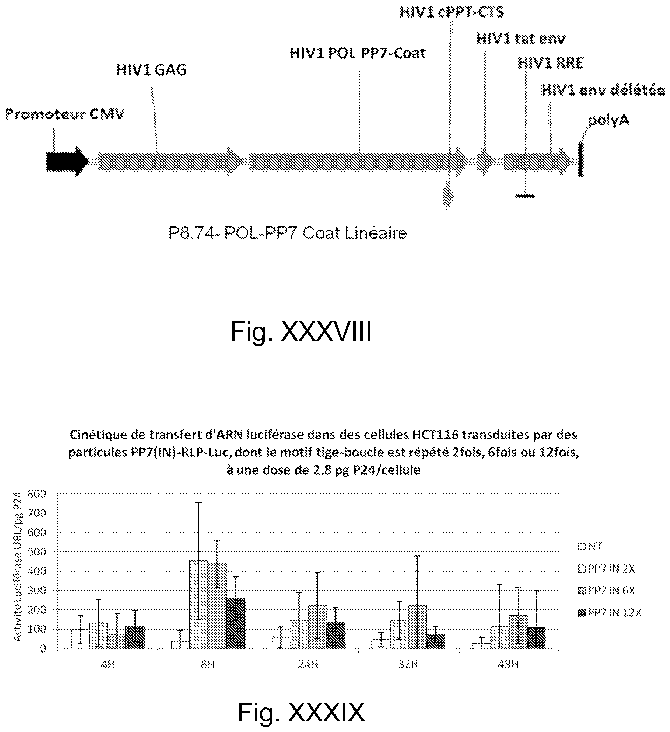

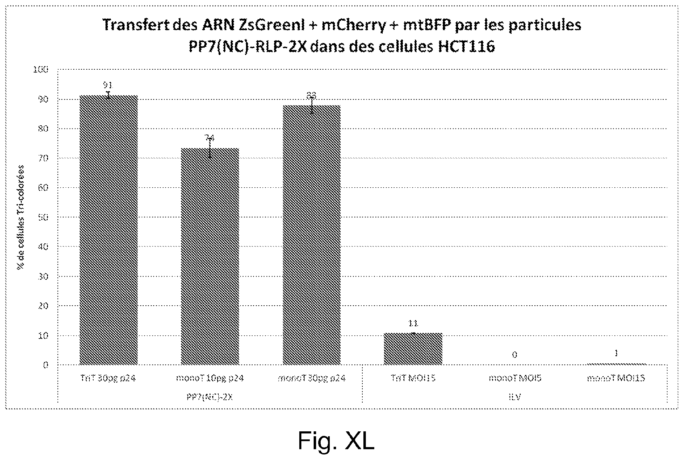

L'invention sera mieux comprise au vu des exemples qui suivent donnés à titre illustratif, avec référence aux figures, qui représentent respectivement :
- La figure I est un schéma illustrant la modification du plasmide d'encapsidation lentiviral p8.74 afin d'insérer un domaine de liaison dans la séquence de l'intégrase, ce plasmide d'encapsidation étant utilisé pour la production de particules lentivirales selon la présente divulgation ;
- La figure II présente différents schémas de constructions de plasmide d'expression portant comme séquence d'ARN d'intérêt la luciférase (fig. IIa), un rapporteur fluorescent (fig. IIb) ou CRE (fig. IIc), ces plasmides d'expression étant utilisés pour la production de particules lentivirales MS2RLP selon l'invention ;
- La figure III présente un schéma illustrant la modification du plasmide d'encapsidation lentiviral p8.74 afin d'insérer un domaine de liaison MS2 Coat dans la séquence de nucléocapside (IIIa) et la construction plasmidique en résultant (IIIb), ce plasmide d'encapsidation étant utilisé pour la production de particules lentivirales MS2RLP selon l'invention ;
- La figure IV est un schéma de construction d'un plasmide d'enveloppe ;
- La figure V présente différents schémas de constructions de plasmide d'expression lentiviral intégratif portant comme séquence d'intérêt la luciférase (fig. Va), un rapporteur fluorescent (fig. Vb) ou CRE (fig. Vc) ;
- La figure VI est un schéma de construction d'un plasmide d'encapsidation, ce plasmide d'encapsidation étant utilisé pour la production de vecteurs lentiviraux intégratifs (ILV) ;
- La figure VII est un schéma de construction d'un plasmide d'encapsidation, ce plasmide d'encapsidation étant utilisé pour la production de vecteurs lentiviraux déficients pour l'intégration (IDLV) ;
- La figure VIII illustre la cinétique d'expression de ZsGreenl dans des cellules HCT116 transduites par des vecteurs lentiviraux (ILV, IDLV) et des particules lentivirales MS2RLP selon l'invention ;
- La figure IX illustre la cinétique d'expression de la luciférase dans des cellules HCT116 transduites par des vecteurs lentiviraux (ILV, IDLV) et des particules lentivirales MS2RLP selon l'invention et transfectées par un plasmide d'expression de la luciférase (pLuc) ;
- La figure X illustre l'effet dose de l'expression de la luciférase après transduction de fibroblastes Foreskin par des vecteurs ILV et des particules lentivirales MS2RLP selon l'invention ;
- La figure XI illustre l'impact de la pureté (production avec ou sans sérum) d'une composition de particules lentivirales MS2RLP selon l'invention, sur la transduction de fibroblastes Foreskin ;
- La figure XII illustre l'impact de la pureté (production avec ou sans sérum) d'une composition de particules lentivirales MS2RLP selon l'invention, sur la transduction de cellules HCT116 ;
- La figure XIII illustre l'impact de la présence de BX795 lors de la transduction de fibroblastes Foreskin par des particules lentivirales MS2RLP selon l'invention et des vecteurs ILV ;
- La figure XIV illustre l'impact de la présence de BX795 lors de la transduction de cellules HCT116 par des particules lentivirales MS2RLP selon l'invention et des vecteurs ILV ;
- Les figures XV, XVI et XVII illustrent la cinétique d'expression de la luciférase par bioluminescence *in vivo,* chez la souris, après injection d'une suspension purifiée ou non de particules MS2RLP selon l'invention et d'une suspension purifiée de vecteurs ILV, la figure XV présente des photographies de chaque animal à des temps donnés, les figures XVI et XVII étant des graphiques représentant les mesures d'expression de la luciférase, à ces mêmes temps ;
- La figure XVIII est un diagramme illustrant l'extinction de la fluorescence chez des cellules HCT116-Lox-dsRed-Lox transduites avec des vecteurs ILV ou des particules MS2RLP selon l'invention ou transfectées avec des plasmides pCre, permettant l'expression de la recombinase Cre ;
- La figure XIX est un diagramme illustrant la quantification du nombre de copie d'ADN Cre dans les cellules HCT116-Lox-dsRed-Lox après transduction par des vecteurs ILV ou des particules MS2RLP selon l'invention ou transfectées avec des plasmides pCre ;
- La figure XX illustre la cinétique de transfert de plusieurs ARN (ARN ZsGreenl + mCherry + mtBFP) dans des cellules HCT116 avec des particules MS2RLP selon l'invention ;
- La figure XXI présente une comparaison de la cinétique de transfert de plusieurs ARN (ARN ZsGreenl + mCherry + mtBFP) dans des cellules HCT116 avec des particules MS2RLP selon l'invention ou des vecteurs IDLV ;
- La figure XXII illustre la cinétique de transfert de plusieurs ARN (ARN ZsGreenl + mCherry + mtBFP) dans des cellules HCT116 avec des vecteurs intégratifs ILV ;
- La figure XXIII illustre l'impact du procédé de production de particules MS2(NC)-RLP-12X ZsGreenl dans des cellules HCT116 à 48h post-transfection ;
- La figure XXIV présente le schéma du plasmide d'expression portant comme séquence d'ARN d'intérêt la luciférase utilisé pour la production de particules lentivirales MS2 (NC)-RLP 2X, comprenant le motif tige-boucle MS2 répété 2 fois, selon l'invention ;
- La figure XXV présente le schéma du plasmide d'expression portant comme séquence d'ARN d'intérêt la luciférase utilisé pour la production de particules lentivirales MS2 (NC)-RLP 6X, comprenant le motif tige-boucle MS2 répété 6 fois, selon l'invention ;
- La figure XXVI présente le schéma du plasmide d'expression portant comme séquence d'ARN d'intérêt la luciférase utilisé pour la production de particules lentivirales MS2RLP, comprenant le motif tige-boucle MS2 répété 12 fois, selon l'invention ;
- La figure XXVII illustre la cinétique de transfert d'ARN Luciférase dans des cellules HCT116 transduites par des particules lentivirales MS2 (NC)-RLP-Luc, comprenant le motif tige-boucle MS2 répété 2 fois, 6 fois ou 12 fois, selon l'invention, à une dose de 10pg p24/cellule ;
- La figure XXVIII présente un schéma du plasmide d'encapsidation étant utilisé pour la production de particules lentivirales MS2 (IN)-RLP selon la présente divulgation, obtenu par la modification du plasmide d'encapsidation lentiviral p8.74 afin d'insérer un domaine de liaison dans la séquence de l'Intégrase comme décrit en Figure I ;
- La figure XXIX illustre la cinétique de transfert d'ARN Luciférase dans des cellules HCT116 transduites par des particules lentivirales MS2 (IN)-RLP-Luc, comprenant le motif tige-boucle MS2 répété 2 fois, 6 fois ou 12 fois, selon la présente divulgation, à une dose de 5pg p24/cellule ;
- La figure XXX présente le schéma du plasmide d'expression portant comme séquence d'ARN d'intérêt la Luciférase utilisé pour la production de particules lentivirales PP7RLP, comprenant le motif tige-boucle PP7 répété 12 fois, selon l'invention ;
- La figure XXXI présente un schéma illustrant la modification du plasmide d'encapsidation lentiviral p8.74 afin d'insérer un domaine de liaison PP7 Coat dans la séquence de nucléocapside ;
- La figure XXXII présente le schéma du plasmide d'encapsidation étant utilisé pour la production de particules lentivirales PP7 (NC)-RLP selon l'invention ;
- La figure XXXIII illustre l'effet dose des particules PP7 (NC)-RLP-Luc 12X selon l'invention sur la transduction des cellules HCT116 ;
- La figure XXXIV présente un schéma du plasmide d'expression avec le motif tige-boucle PP7 répété 2 fois, comprenant la cassette d'expression de la Luciférase (Figure XXXIVa) ou d'un rapporteur fluorescent (Figure XXXIVb) pour la production de particules PP7 (NC)-RLP 2X selon l'invention ou PP7 (IN)-RLP 2X selon la présente divulgation ;
- La figure XXXV présente un schéma du plasmide d'expression de la Luciférase avec le motif tige-boucle PP7 répété 6 fois pour la production de particules PP7 (NC)-RLP 6X selon l'invention ou PP7 (IN)-RLP 6X selon la présente divulgation ;
- La figure XXXVI illustre la cinétique de transfert d'ARN Luciférase dans des cellules HCT116 transduites par des particules PP7 (NC)-RLP-Luc, dont le motif tige-boucle PP7 est répété 2 fois, 6 fois ou 12 fois, à une dose de 10pg p24/cellule ;
- La figure XXXVII présente un schéma de la modification du plasmide d'encapsidation lentiviral p8.74 afin d'insérer un domaine de liaison dans la séquence de l'Intégrase ;
- La figure XXXVIII présente un schéma du plasmide d'encapsidation utilisé pour la production de particules lentivirales PP7 (IN)-RLP selon la présente divulgation, obtenu par la modification du plasmide d'encapsidation lentiviral p8.74 présenté Figure XXXVII ;
- La figure XXXIX illustre la cinétique de transfert d'ARN Luciférase dans des cellules HCT116 transduites par des particules lentivirales PP7 (IN)-RLP-Luc, comprenant le motif tige-boucle PP7 répété 2 fois, 6 fois ou 12 fois, selon la présente divulgation, à une dose de 2,8 pg p24/cellule ; et
- La figure XL illustre le transfert des ARN ZsGreenl + mCherry + mtBFP par les particules PP7 (NC)-RLP 2X dans les cellules HCT116.

### EXEMPLE 1 : Construction des particules lentivirales MS2RLP

### I. Matériel & Méthodes

### 1. Constructions des plasmides

### 1.1 Plasmides pour la production de particules lentivirales MS2RLP

- **Plasmide d'expression d'une séquence d'intérêt** : Le plasmide d'expression est porteur d'une cassette d'expression promoteur-séquence d'intérêt-polyA (voir Figure II) avec ou non une séquence intronique ou stabilisatrice d'ARN. Afin de véhiculer les ARNm dans les particules lentivirales, 12 répétitions du motif tige-boucle de l'ARN MS2 (ctagaaaacatgaggatcacccatgtctgcag, SEQ ID N°1) ont été insérées au sein d'une cassette d'expression en aval du gène rapporteur. Le promoteur utilisé peut être celui du CMV (Figure IIa) ou EF1 (Figures IIb et IIc) mais d'autres promoteurs peuvent être utilisés. La séquence d'intérêt peut être un ADN codant une protéine reportrice comme la luciférase Firefly native (Figure IIa), une protéine fluorescente (Figure IIb) verte (ZsGreenl), rouge (mCherry) ou bleue (mtBFP), ou un ADNc codant une protéine, par exemple la protéine CRE (Figure IIc). La séquence d'intérêt peut également être celle d'un shRNA, d'un miRNA, d'un sgRNA, d'un LncRNA ou d'un circRNA.
- **Plasmide d'encapsidation** : La particule lentivirale a été modifiée pour contenir au sein de la protéine de nucléocapside, en lieu et place du second domaine en doigt de Zn, la séquence de la protéine « Coat » du bactériophage MS2. Le plasmide d'encapsidation p8.74 (Figure III), porteur des gènes codant les protéines de structures et de fonction (Gag, Pol), utilisé pour la production des particules MS2RLP est modifié selon la stratégie illustrée par la Figure IIIa : ce plasmide p8.74 est utilisé pour générer, par PCR d'assemblage, un plasmide dépourvu du second doigt de zinc de la protéine de nucléocapside p8.74ΔZF. Le second doigt de zinc est substitué par la protéine Coat du phage MS2 par clonage Hpal, pour générer le plasmide p8.74ΔZF-MS2-Coat. On obtient ainsi la construction illustrée en Figure IIIb. La séquence codant Pol peut être délétée ou mutée dans certains éléments de fonction comme par exemple la séquence codant la transcriptase inverse (RT) ou l'intégrase (IN) sans altérer la fonction des MS2RLP.
- **Plasmide de l'enveloppe (pENV)** : Ce plasmide est porteur du gène codant une protéine d'enveloppe, qui peut être la VSVG codant la protéine d'enveloppe du Virus de la stomatite vésiculaire (Figure IV).

### 1.2 Plasmides pour la production de vecteurs lentiviraux intégratifs ILV

- **Plasmide d'expression d'une séquence d'intérêt :** Le plasmide d'expression est porteur d'une cassette d'expression promoteur-séquence d'intérêt (Figure V). Ce plasmide peut contenir d'autres éléments comme la séquence native WPRE (Woodchuck Hepatitis Virus Posttranscriptional Regulatory Element) ou encore la séquence cPPT/CTS. La pathogénicité virale est éliminée par la substitution de régions du génome viral requises pour la réplication rétrovirale par le transgène.
- **Plasmide d'encapsidation :** Le plasmide d'encapsidation p8.74, porteur des gènes codant les protéines de structures et de fonction (Gag, Pol) est utilisé pour la production des vecteurs lentiviraux intégratifs (Figure VI).
- **Plasmide de l'enveloppe (pENV)** : Ce plasmide est identique au plasmide d'enveloppe utilisé pour la production de particules lentivirales MS2RLP (Figure IV).

### 1.3 Plasmides pour la production de vecteurs lentiviraux déficients pour l'intégration IDLV : mutant D64L

Les 3 plasmides sont les mêmes que ceux décrits au point 1.2 sauf pour le plasmide d'encapsidation qui comprend une mutation D64L de la séquence *pol* codant pour l'intégrase (Figure VII). Cette mutation sur un seul acide aminé induit une inhibition de l'intégration (Nightingale et al., 2006 et Apolonia et al., 2007). La mutation D64L sur l'intégrase (IN) a été introduite dans le plasmide d'encapsidation p8.74 par mutagénèse dirigée. La mutation a été vérifiée par séquençage.

### 1.4 Plasmides pLuc et pCre

Ces plasmides d'expression sont similaires à ceux utilisés pour la production des vecteurs ILV et IDLV (Figure Vb pour pLuc et Figure Vc pour pCre), utilisé directement en transfection pour contrôle.

### 2. Productions des lots

Après la transfection des plasmides sur des cellules, les surnageants sont récoltés et utilisés bruts ou concentrés/purifiés selon le procédé décrit dans la demande WO 2013/014537.

### 2.1 Production des vecteurs lentiviraux et particules lentivirales

Les productions sont réalisées en CellSTACK 10 étages (6360 cm², Corning) avec des cellules HEK293T (ATCC, CRL-11268), cultivées dans Dulbecco's Modified Eagle's Medium (DMEM, Gibco, Paisley, UK) supplémenté par 1% penicillin/streptomycin et 1% d'ultraglutamine (PAA) à 37°C en atmosphère humide à 5% CO_{2.} Pour l'expérience d'étude de l'impact du sérum, le DMEM est supplémenté avec 10% de SVF. En particulier, aucune induction au sodium butyrate n'est effectuée. Pour chaque lot (MS2RLP, ILV et IDLV), le mélange de transfection est composé des trois plasmides suivants :
- Un des plasmides d'expression décrit ci-avant, selon que l'on forme une particule (MS2RLP) ou un vecteur (ILV, IDLV),
- p8.74ΔZF Coat (MS2RLP), p8.74 (ILV) ou p8.74 muté à la position D64L (IDLV), et
- pENV portant l'enveloppe VSV-G.

24 heures après transfection standard au phosphate de calcium, le surnageant de culture est remplacé par du milieu DMEM frais et non supplémenté. Les cellules sont incubées à 37°C / 5% CO2. Après changement du milieu, le surnageant est collecté quatre fois (32h, 48h, 56h et 72h post transfection). Chaque récupération est clarifiée par centrifugation 5min à 3000g avant d'être microfiltrée sur filtre de 0,45µm (Stericup, Millipore). Toutes les récupérations sont alors mélangées pour composer le surnageant brut.

### 2.2 Concentration et purification des vecteurs lentiviraux et particules lentivirales

Les vecteurs et particules sont concentrés et purifiés selon l'une des deux procédés ci-après :
- Le procédé P1 vise à réaliser une ultrafiltration frontale du surnageant sur des unités centrales de centrifugation.
- Le procédé P2 vise à réaliser une ultrafiltration tangentielle puis une diafiltration du surnageant. Le surnageant brut est concentré et purifié par ultrafiltration tangentielle via l'utilisation de cartouches de fibres creuses en polysulfone. Le surnageant est traité par diafiltration pour 20 diavolumes en mode continu contre du tampon DMEM ou TSSM. Après la diafiltration, le rétentat est récupéré puis concentré à nouveau par ultrafiltration frontale sur des unités centrales de centrifugation.

### 3. Titration

### 3.1 Titration des particules fonctionnelles par qPCR.

Les cellules HCT116 (ATCC, CCL-247) sont ensemencées en plaque 96 puits dans 100µL de DMEM supplémenté de 10% SVF, 100 µg/mL Stretomycin, 100 U/mL Penicillin et 2mM L-Gln puis incubées 24h à 37°C / 5% CO2. Six dilutions sériées sont réalisées pour chaque vecteur ainsi que pour un étalon interne. Les cellules sont transduites par ces dilutions sériées en présence de Polybrene® 8µg/mL (Sigma) puis incubées trois jours à 37°C / 5% CO2. Pour chaque série d'échantillons, un puits de cellules non transduites est rajouté en contrôle. Les cellules sont ensuite trypsinées et le titre (Unité de Transduction/mL) est déterminé par qPCR après extraction de l'ADN génomique en utilisant le Nucleospin tissue gDNA extraction kit (Macherey-Nagel). Le titre obtenu (TU/mL) par qPCR est normalisé par l'étalon interne dont le titre a été précédemment déterminé par FACS.

### 3.2 Quantification des particules physiques par test ELISA p24.

La protéine de capside p24 est détectée directement sur le surnageant viral en utilisant et en suivant les recommandations du kit HIV-1 p24 ELISA kit (Perkin Elmer). La protéine p24 capturée est complexée avec un anticorps polyclonal biotinylé, puis détectée par une streptavidine conjuguée à la péroxydase HRP. Le complexe résultant est détecté par spectrophotométrie après incubation avec le substrat ortho-phenylenediamine-HCl (OPD) produisant une coloration jaune qui est directement proportionnelle à la quantité de protéine p24 capturée. L'absorbance de chaque puits est quantifiée au lecteur de plaque Synergy H1 Hybrid (Biotek) et calibrée contre l'absorbance d'une gamme étalon de protéine p24. Le titre viral exprimé en particules physiques par mL est calculé à partir de la concentration de protéine p24 obtenue sachant que 1pg de protéine p24 correspond à 10⁴ particules physiques.

### 4. Monotransduction

Cet exemple vise à mettre au point les conditions de la monotransduction par les vecteurs lentiviraux ou les particules lentivirales produites ci-avant.

### 5. Cinétique d'expression de la ZsGreenl

Les cellules HCT116 ensemencées la veille à 25000 cellules/cm2 en plaque 24 puits (Corning) sont transduites à 100000 PP/cellule en présence de 8 µg/mL de Polybrene®. A 8h et 24h post-transduction, les cellules sont trypsinées et analysées par cytométrie en flux pour mesurer le pourcentage de cellules fluorescentes. Chaque expérience est réalisée en triplicat.

### 6. Cinétique d'expression de la luciférase

### 6.1 Cellules HCT116

Les cellules HCT116 (ATCC, CCL-247) sont ensemencées en plaque 6 ou 24 puits et incubées 24h à 37°C / 5% CO2. La transduction par les vecteurs ILV, IDLV ou les particules MS2RLP est réalisée en présence de 4µg/mL Polybrene. Le surnageant de transduction est éliminé 4 heures après et remplacé par du milieu de culture supplémenté frais. De 4h à 24h post-transduction, les cellules sont récupérées et l'expression de la luciférase est analysée à l'aide du kit OneGlo Luciferase assay (Promega) en suivant les recommandations du fournisseur et en utilisant le lecteur de plaque Synergy H1 Hybrid (Biotek). Ce test est réalisé en triplicat. En parallèle, les cellules HCT116 sont transfectées par le plasmide pLuc. Pour cela, 50000 cellules HCT116 sont transfectées par 2,6 µg du plasmide pLuc à l'aide du PEI PRO® (Polyplus Transfection).

### 6.2 Fibroblastes Foreskin

Des fibroblastes Foreskin, ensemencés la veille à 10 000 cellules/cm² en plaque 6 puits (Corning) sont transduits en présence de 4µg/mL de Polybrene®. Les cellules ont été transduites à 200 000 et 500 000PP/cellule avec les MS2RLP-Luc, ou à MOI5 avec un vecteur lentiviral intégratif Luc.

Les cellules transduites par les MS2RLP-Luc sont trypsinées 8h post-transduction et passées en plaque 96 puits, opaque fond noir dans 100µL de milieu complet. Les cellules transduites par les ILV Luc sont trypsinées 24h post-transduction et passées en plaque 96 puits, opaque fond noir dans 100µL de milieu complet. 3 minutes avant lecture au luminomètre, 100µL de réactif One Glo Luciferase (Promega) sont ajoutés par puits à analyser.

### 6.3 Chez la souris

Les mesures de cinétique d'expression de la luciférase par bioluminescence *in vivo* ont été réalisées.

Trois groupes de souris mâles Balb/c sont comparés, un premier groupe a reçu une suspension de vecteur lentiviral de type intégratif rLV-EF1-Luc purifiée (n=2), un second groupe a reçu une suspension de particules MS2RLP-Luc (n=4) produites selon le procédé P2, un dernier groupe a reçu une suspension de particules MS2RLP-Luc (n=4) produites selon le procédé P1. Un animal non injecté a servi de contrôle négatif pour la détermination du bruit de fond.

Les mesures sont effectuées de 5h à 24h (Figure XV A), et de 48h à 168h (Figure XV B) après injection systémique. Chaque mesure de l'expression de la luciférase a été effectuée 15 minutes après l'injection intra-péritonéale de 300 mg / kg de D-luciférine (Promega) avec une caméra Andor, et le logiciel d'acquisition d'images Solis. Le temps d'acquisition est de 5 minutes et les images résultantes ont été traitées et normalisé avec le logiciel ImageJ. La Figure XV montre les images de chaque animal aux temps donnés. Les graphiques (Figures XVI et XVII) représentent les mesures de l'expression de la luciférase, en unités relatives de luminescence (RLU) à ces mêmes temps.

### 7. Impact de la pureté des particules pour la transduction

### 7.1 Fibroblastes Foreskin

Des fibroblastes Foreskin ensemencés la veille à 10000 cellules/cm² en plaque 24 puits CellBind (Corning) sont transduits en présence de 4µg/mL de Polybrene®. Les cellules ont été transduites à 500 000 PP/cellule avec deux types de qualité de surnageants : lot produit en présence sérum puis obtenu selon le procédé P2 vs. lot produit sans sérum puis obtenu selon le procédé P2. A 8h post-transduction, les cellules sont trypsinées et passées en plaque 96 puits, opaque fond noir dans 100µL de milieu complet. 3 minutes avant lecture au luminomètre, 100µL de réactif One Glo Luciferase (Promega) sont ajoutés par puits à analyser.

### 7.2 Cellules HCT116

Les cellules ensemencées la veille à 25000 cellules/cm² en plaque 24 puits CellBind (Corning) sont transduites en présence de 4µg/mL de Polybrene®. Les cellules ont été transduites à 100000 PP/cellule avec deux types de qualité de surnageants : lot produit en présence sérum puis obtenu selon le procédé P1 vs. lot produit sans sérum puis obtenu selon le procédé P1. A 8h post-transduction, les cellules sont trypsinées et passées en plaque 96 puits, opaque fond noir dans 100µL de milieu complet. 3 minutes avant lecture au luminomètre, 100µL de réactif One Glo Luciferase (Promega) sont ajoutés par puits à analyser.

### 8. Impact du BX795

### 8.1 Fibroblastes Foreskin

Des fibroblastes Foreskin ensemencés la veille à 10 000 cellules/cm² en plaque 6 puits CellBind (Corning) sont transduits en présence de 4µg/mL de Polybrene®. Les cellules ont été transduites à 500000 PP/Cellule pour les particules MS2RLP, et MOI 5 pour les ILV. A 8h post-transduction, les cellules sont trypsinées et passées en plaque 96 puits, opaque fond noir dans 100µL de milieu complet. 3 minutes avant lecture au luminomètre. 100µL de réactif One Glo Luciferase (Promega) sont ajoutés par puits à analyser.

### 8.2 Cellules HCT116

Des cellules HCT116 ensemencées la veille à 25 000 cellules/cm² en plaque 6 puits CellBind (Corning) sont transduites en présence de 4µg/mL de Polybrene®. Les cellules ont été transduites à 100000 PP/Cellule pour les particules MS2RLP, et MOI 5 pour les ILV. A 8h post-transduction, les cellules sont trypsinées et passées en plaque 96 puits, opaque fond noir dans 100µL de milieu complet. 3 minutes avant lecture au luminomètre 100µL de réactif One Glo Luciferase (Promega) sont ajoutés par puits à analyser.

### 9. Expression in vitro de la recombinase CRE

Dans un premier temps, des cellules HCT116 (ATCC, CCL-247) sont transduites par le vecteur lentiviral ILV-EF1-Lox-dsRed-Lox à MOI20 en présence de 4µg/mL Polybrene. Six jours plus tard, cette population polyclonale est clonée par dilution limite en ensemençant les cellules en plaque 96 puits à raison de 0,5 cellules par puits. La lignée monoclonale HCT116-lox-dsRed-lox-clone 14 a été sélectionnée par cytométrie (MACS Quant VYB, Miltenyi), sur la base du niveau d'expression de la dsRed.

Dans un second temps, les lignées cellulaires polyclonales et monoclonales ont été transduites par les vecteurs ILV-Cre à MOI 5 ou les particules MS2RLP-Cre à une dose de 2⁵ Particules Physiques (PP)/cellule, en présence de 4µg/mL de Polybrene®.

Les cellules polyclonale et monoclonale HCT116-Lox-dsRed-Lox transduites par les particules MS2RLP-Cre ou par les vecteurs ILV-Cre, ont été incubées à 37°C / 5% CO2 pendant 14 jours. 14 jours post-transduction, l'expression de la dsRed est analysée par cytométrie en flux. Des cellules non transduites (NT) servent de contrôle. Cette expérience est réalisée en triplicat. En parallèle, les cellules polyclonale et monoclonale HCT116-Lox-dsRed-Lox ont été transfectées par le plasmide pCre. Pour cela, 50000 cellules HCT116-Lox-dsRed-Lox ont été transfectées par 2,6 µg du plasmide pCre à l'aide du PEI PRO ® (Polyplus Transfection).

Après extraction de l'ADN des cellules HCT116-Lox-dsRed-Lox transduites par les vecteurs ILV-Cre ou par les particules MS2RLP-Cre, ou transfectées par le plasmide pCRE, le nombre de copies intégrées dans les cellules est mesuré par qPCR par détection d'une courte séquence de la recombinase Cre :
gcatttctggggattgcttataacaccctgttacgtatagccgaaattgccaggatcagggttaaagatatctca cgtactgacggtgggagaat (SEQ ID N°2)
avec le couple d'oligonucléotides de qPCR suivants :
   Q-CRE-F: 5'- GCATTTCTGGGGATTGCTTA-3' (SEQ ID N°3)
   Q-CRE-R: 5'- ATTCTCCCACCGTCAGTACG-3' (SEQ ID N°4).

### II. Résultats

### 1. Niveaux de production des particules lentivirales

Les premières expériences ont consisté à comparer les niveaux de production respectifs des particules MS2RLP, des vecteurs IDLV ou ILV. Des lots de chaque type de particules ont été produits et les titres des particules ont été mesurés par un test Elisa p24. Les résultats sont présentés dans le Tableau 1 ci-après :

**Tableau 1 : Titres des vecteurs ILV, IDLV-D64L et des particules MS2RLP**

| Type de particules | N° lot | Titres des surnageants PP/mL |
|---|---|---|
| MS2RLP-Luc | rV2.1A1.1740 C2 | 5,5.10¹¹ |
| IDLV-Luc | rV2.1A1.1732 C2 | 3,1.10¹¹ |
| ILV-Luc | rV2.1A1.2055 C2 | 6,9.10¹¹ |

Ces résultats montrent que les trois types de particules présentent des titres équivalents (PP/ml +/-) après concentration par la même technique d'ultrafiltration tangentielle. Les modifications d'encapsidation ou la mutation de l'intégrase ne conduisent donc pas à un déficit de production des particules lentivirales.

### 2. Cinétique d'expression dans les cellules cibles.

Une première série de tests a été réalisée avec des particules MS2RLP porteuses d'un seul type d'ARN codant une protéine fluorescente verte (ZsGreenl) caractérisée par un temps de demi-vie long. Les résultats obtenus avec des particules MS2RLP sont présentés sur la Figure VIII et montrent que le transfert des ARN est détecté de façon précoce (dès 8h post-transduction). Les résultats obtenus montrent un pourcentage de cellules HCT116 positives comparable à 24 heures par les particules lentivirales MS2RLP ou les vecteurs lentiviraux ILV ou IDLV-D64L. Le pourcentage de cellules positives est détectable dès 8 heures pour les particules MS2RLP mais seulement à partir de 24 heures pour les vecteurs ILV et IDLV-D64L. Ces particules MS2RLP sont donc fonctionnelles et présentent une capacité équivalente à celle d'un ILV ou d'un IDLV-D64L pour introduire une séquence codante dans une population cellulaire.

Dans le cas d'un système lentiviral intégratif, les ARNs sont rétrotranscrits par la RT. L'ADNc est alors transloqué dans le noyau avant son intégration dans le génome de la cellule hôte. C'est à partir de cette étape seulement que la transcription puis la traduction du transgène ont lieu. Dans le cas d'un système lentiviral non-intégratif (mutation sur l'IN), les ARNs sont rétrotranscrits par la RT. L'ADNc est alors transloqué dans le noyau avant d'être transcrit en ARN puis traduit en protéine.

Seules les particules MS2RLP permettent une expression précoce du transgène du fait de l'absence de toutes ces étapes intermédiaires. L'ARN délivré est directement traduit en protéine par la machinerie cellulaire.

Une deuxième série de tests a été réalisée avec des particules MS2RLP porteuses d'un seul type d'ARN codant la Luciférase (Luc) caractérisée par un temps de demi-vie court. Les résultats obtenus avec des particules MS2RLP sont présentés sur la Figure IX et montrent que l'activité optimale de la Luciférase est également détectée de façon précoce.

L'expression de la Luciferase est transitoire car elle diminue progressivement de 8h à 24h. En revanche, l'expression de la fluorescence reste détectable voire stable sur ce type de cinétique. Ces résultats doivent considérer le temps de demi-vie de la protéine rapporteur utilisée. Il est évident que la détection du gène rapporteur est proportionnelle à ce temps de demi-vie. Plus ce dernier sera long, plus l'activité liée à la protéine sera longue.

Sur la Figure X, on peut également observer que l'intensité d'expression de la luciférase varie en fonction du ratio PP/cellule utilisé lors de la transduction. Cet effet dose est montré notamment sur les fibroblastes Foreskin.

Il est donc important de pouvoir atteindre de hautes doses de particules MS2RLP à appliquer sur les cellules à transduire pour induire un niveau d'expression significatif capable de se traduire par une activité biologique. La concentration des particules apparait comme un facteur clé de succès. Dans des fibroblastes Foreskin, l'expression de Luciférase induite avec des particules à haute dose soit 500000 PP/cellule, est équivalente à celle obtenue avec des vecteurs lentiviraux intégratifs classiques à une multiplicité d'infection (MOI) de 5. La détermination de la dose optimale pour la transduction des HCT116 par les particules MS2RLP a été réalisée dans les mêmes conditions que pour les fibroblastes Foreskin, en testant les doses de 50000, 100000 et 200000 PP/Cellule. La dose retenue est 100000 PP/cellule, pour laquelle on obtient le meilleur transfert d'ARN Luciferase. Ces résultats montrent que la méthode de transduction doit être adaptée en fonction de la permissivité des cellules cibles.

Ces résultats sont confirmés par des expériences d'injection *in vivo* des particules MS2RLP-Luc qui montrent une détection de l'expression de la luciférase 5 heures après injection.

### 3. Impact de la production et de la pureté de la suspension de particules MS2RLP sur l'efficacité de transduction.

Selon le type cellulaire cible, il est important de pouvoir augmenter la dose de particules afin d'obtenir un nombre important de cellules exprimant les ARN transportés. Il est possible d'augmenter la dose de particules sans affecter la viabilité des cellules cibles en utilisant des particules purifiées, comme montré dans la demande WO 2013/014537. En effet, la pureté finale du lot de particules lentivirales est affectée par la présence de sérum de veau fœtal (SVF).

Pour évaluer l'impact de la pureté de la suspension de particules MS2RLP sur l'expression de la Luciférase, nous avons comparé l'expression de la Luciférase en fonction de la pureté de deux types de lots de surnageant :
- Un surnageant concentré à partir de particules MS2RLP produites avec sérum,
- Un surnageant concentré à partir de particules MS2RLP produites sans sérum.

Cette étude a été réalisée à la fois dans des cellules immortalisées et permissives HCT116 (Figure XII) et dans des cellules primaires moins permissives et délicates, les fibroblastes Foreskin (Figure XI).

Les résultats obtenus montrent que la pureté du lot a un impact important sur l'efficacité de transduction de cellules primaires délicates à haute dose. A 500000 PP/cellule, l'effet de la pureté est considérable puisque l'activité de la Luciférase est augmentée de près de 80% avec le lot produit sans sérum (Figure XI). En présence de SVF, il y a en revanche une diminution de l'expression de la luminescence.

Sur des cellules immortalisées HCT116 très résistantes, l'impact de la pureté n'est pas détectable sur l'activité Luciférase (Figure XII). En revanche, le lot produit en présence de sérum présente des résultats plus hétérogènes, ce qui montre une reproductibilité beaucoup plus faible qu'avec le lot produit sans sérum. Ce résultat justifie donc le choix de produire des lots de particules en absence de sérum.

Ces expériences démontrent d'une part la nécessité de concentrer ces particules lentivirales pour obtenir un niveau d'expression important compatible avec l'induction d'un phénotype au niveau cellulaire et tissulaire et d'autre part l'impact de la pureté de ces lots sur l'efficacité du transfert de gènes.

### 4. Identification des conditions optimales de transfert de gènes par les particules MS2RLP.

Pour augmenter l'efficacité de transfert d'ARN, les résultats obtenus par Sutlu et al. (2012) pour augmenter l'efficacité de transduction de cellules Natural Killer NK ont été transposés. Plus particulièrement, l'hypothèse a été émise que les réponses antivirales basées sur le récepteur Toll-like receptor (TLR) et / ou RIG-I-like (RLR) pouvaient restreindre l'efficacité de transfert d'ARN dans certaines cellules. Afin de tester cette hypothèse, un inhibiteur de petites molécules de signalisation TLR et RLR a été utilisé pendant la mise en contact des cellules avec les particules MS2RLP. La molécule BX795 est un inhibiteur du complexe TBK1/IKKε, qui agit en tant que médiateur commun dans les voies de signalisation de RIG-I, MDA-5, et TLR3.

L'utilisation de la molécule BX795 à une concentration de 6 µM augmente considérablement l'efficacité de transduction des cellules HCT116 (Figure XIV) et des fibroblastes Foreskin (Figure XIII). Les temps d'observation (8h pour les particules MS2RLP, 24h pour les ILV) tiennent compte du fonctionnement de chaque particule (Figures VIII et IX). On peut noter un effet synergique entre les particules MS2RLP et l'utilisation de BX795 dans les cellules HCT116 comme dans les fibroblastes Foreskin, alors que le BX795 induit un effet négatif avec les vecteurs lentiviraux intégratifs ILV dans ces deux types cellulaires. Cet effet peut être lié à un différentiel du nombre de molécules d'ARN délivrées par ces deux types de particules.

### 5. Etude du transfert de gènes par des particules MS2RLP Luciferase in vivo.

L'injection *in vivo* de particules MS2RLP-Luc vise à mettre en évidence la cinétique d'expression d'un transgène, dans le cas présent la luciférase, dans les différents organes de souris injectées par voie systémique. Une suspension de vecteurs intégratifs ILV-EF1-Luc purifiée est utilisée comme contrôle positif. Les mesures sont faites dès 5h et jusqu'à 7 jours post injection. L'administration par voie intraveineuse engendre une dilution de la suspension injectée et donc une diffusion du signal de bioluminescence qu'il faut considérer. Les analyses sont donc faites sur l'animal entier afin de prendre en compte la répartition du signal dans l'intégralité de l'organisme. Une suspension de particules MS2RLP-Luc produite selon le procédé P1 en présence de sérum et une suspension de particules MS2RLP-Luc purifiée selon le procédé P2 sont testées. L'injection de la suspension de particules MS2RLP-Luc, selon le procédé P1 en présence de sérum dans lequel la purification est moins poussée, a entraîné des difficultés à la fois de prélèvement et d'injection. En effet, cette suspension de particules MS2RLP-Luc, obtenue par le procédé P1 en présence de sérum, se caractérise par une suspension brune à forte viscosité qui est particulièrement difficile à prélever avec une aiguille de 29G utilisée pour des injections *in vivo.* Cette difficulté se retrouve au moment de la délivrance de la suspension dans la veine de la queue. L'utilisation de suspension de particules MS2RLP-Luc obtenue par un procédé P1 en présence de sérum a pour conséquence une mauvaise administration des particules qui se retrouvent localisées dans la zone caudale de l'animal (i.e. au site d'injection) et ne passent pas dans la circulation générale de l'animal (Figure XV A), temps H5 et H8 du groupe «MS2RLP-Luc produit selon le procédé P1 en présence de sérum »). La suspension de particules MS2RLP-Luc obtenue par un procédé P1 en présence de sérum ne peut donc pas être utilisée dans le cadre d'études *in vivo.*

La suspension purifiée de particules MS2RLP-Luc a pu être normalement administrée et donne lieu à une expression de la luciférase de 818 ULR à 5 heures après injection. Ce signal est toujours visible à 8 heures puis disparait aux temps ultérieurs (Figures XV A, XVI et XVII), groupe « MS2RLP-Luc purifié »). Le signal de bioluminescence se retrouve principalement dans le foie et la rate (Figure XV A), groupe «MS2RLP-Luc purifié »). Par comparaison, à 5 heures, la luminescence mesurée chez les animaux ayant reçu la suspension purifiée de vecteurs viraux intégratifs ILV-EF1-Luc n'est pas significative puisque la transcription inverse et l'intégration sont abortives à ces temps courts. En revanche, à un stade précoce (5 heures post injection), le signal obtenu avec la suspension purifiée de particules MS2RLP-Luc est significatif et a atteint un niveau d'expression deux fois plus important que le signal obtenu avec la suspension purifiée de vecteurs viraux intégratifs ILV-EF1-Luc. La suspension purifiée de particules MS2RLP-Luc atteint au bout de 5 heures un niveau d'expression équivalent à celui de la suspension purifiée de vecteurs viraux intégratifs ILV-EF1-Luc au bout de 168h (Figures XV B et XVI) groupe « ILV-EF1-Luc purifié »). La suspension purifiée de particules MS2RLP-Luc permet d'obtenir *in vivo* une expression transitoire optimisée et équivalente à un niveau d'expression résultant d'un mécanisme intégratif de transduction lentivirale. L'expression est transitoire du fait de la non-intégration du transgène dans le génome des cellules. Le procédé de concentration/purification permet donc d'obtenir des suspensions de particules MS2RLP efficaces après injection *in vivo.*

### 6. Comparaison de la fonctionnalité des différentes particules

Les cellules HCT116-Lox-dsRed-Lox ont été préalablement générées par transduction avec un vecteur lentiviral intégratif portant la séquence suivante: EF1-Lox-DsRed-Lox. Après obtention d'une lignée fluorescente polyclonale, une lignée monoclonale a été obtenue par dilution limite. Il est à noter que le nombre de copies de la séquence Lox-DsRed-Lox intégrées a été quantifié par qPCR dans les lignées polyclonale et monoclonale. Le nombre de copies intégrées est en moyenne de 6 pour la lignée polyclonale et de 13 copies pour la lignée monoclonale.

Ces deux lignées ont été transduites par des particules MS2RLP porteuses de la séquence codante pour l'enzyme recombinase Cre. En parallèle, ces cellules HCT116-Lox-dsRed-Lox ont été transduites par des vecteurs lentiviraux ILV porteurs de la même séquence Cre.

Les cellules polyclonale et monoclonale HCT116-Lox-dsRed-Lox transduites par les particules MS2RLP-Cre, les vecteurs ILV-Cre ont été incubées à 37°C / 5% CO2 pendant 14 jours avant analyse au cytomètre. Les résultats de quantification du pourcentage de cellules fluorescentes par cytométrie en flux sont présentés sur la Figure XVIII. Les résultats montrent une quasi-disparition de la fluorescence au sein des populations polyclonale et monoclonale mises en contact avec les particules MS2RLP-Cre. En effet, le pourcentage de cellules fluorescentes est passé de 100% à moins de 10%. Le résultat est beaucoup moins efficace avec les vecteurs intégratifs ILV-Cre (15% de diminution) ainsi que dans la condition de transfection du plasmide pCre (aucune diminution).

Les événements d'intégration après transfert des acides nucléiques codant la recombinase CRE dans des cellules HCT116 par des particules MS2RLP-Cre, par des vecteurs ILV-Cre, ou par des plasmides pCre ont été vérifiés. Dans ce but, nous avons quantifié le nombre de copies résiduelles du génome porté par les différents types de particules et plasmides, dans les cellules HCT116-Lox-dsRed-Lox mises en contact avec les vecteurs ILV, les particules MS2RLP ou les plasmides codant la protéine CRE. Dans ce but, les ADN totaux des cellules HCT116 sauvages et modifiées ont été préparés 6 et 14 jours après la transduction et l'ADN codant la recombinase CRE a été mesurée par qPCR.

Les résultats présentés en Figure XIX montrent qu'avec des vecteurs lentiviraux intégratifs le nombre d'événements d'intégration est de 4 copies par cellule à 6 jours. Ce nombre est cohérent avec la multiplicité d'infection (MOI) utilisée. Le contrôle de transfection montre un grand nombre de copies d'ADN à 6 jours, correspondant à la détection du plasmide présent dans les cellules, puis disparition totale des copies d'ADN Cre à 14 jours, du fait de l'absence d'intégration du plasmide dans le génome des cellules cibles.

Avec les particules MS2RLP, aucun événement d'intégration n'est détecté. En effet, l'expression n'étant pas dépendante de la transcriptase inverse ni des extrémités LTR, ces particules délivrent de l'ARN au sein des cellules cibles qui est soit directement traduit en protéines, soit entrainé par une protéine dans le noyau des cellules et ne génère aucune espèce d'ADN.

### EXEMPLE 2 : Transfert de plusieurs ARN différents par transduction unique avec des particules MS2RLP

### I. Matériel & Méthodes

### 1. Mono- et tri-transduction

Cet exemple est réalisé en utilisant des particules MS2RLP ou des vecteurs IDLV permettant soit le transfert d'un seul type d'ARN permettant l'expression d'une seule protéine (ZsGreenl, mCherry ou mtBFP), soit le transfert de plusieurs types d'ARNs permettant l'expression de plusieurs protéines différentes (ZsGreenl + mCherry + mtBFP).

Les particules MS2RLP et les vecteurs lentiviraux non-intégratifs IDLV sont produits dans les conditions suivantes :
- Les particules ont été produites par transfection de cellules HEK293T avec un seul plasmide d'expression codant soit la ZsGreenl, soit la mCherry, soit la mtBFP (comme cela est décrit à l'exemple 1), en plus des plasmides d'encapsidation et de l'enveloppe,
- Les particules ont été produites par transfection de cellules HEK293T avec trois plasmides d'expression codant respectivement la ZsGreenl, la mCherry et la mtBFP ajoutés en quantité équimolaire, pour une quantité totale de plasmides d'expression équivalente à la quantité totale de plasmides d'expression utilisée pour une production de particule avec un seul plasmide d'expression, en plus des plasmides d'encapsidation et de l'enveloppe.

Les cellules HCT116 ont été transduites par les particules MS2RLP soit :
- par transduction avec les 3 types de particules MS2RLP ZsGreenl, mCherry ou mtBFP indépendamment,
- par mono-transduction avec les particules produites avec les trois plasmides d'expression codant ZsGreenl, mCherry et mtBFP simultanément.

En parallèle, des cellules HCT116 sont transduites par des vecteurs IDLV soit :
- par transduction avec les 3 types de vecteurs IDLV ZsGreenl, mCherry ou mtBFP indépendamment,
- par mono-transduction avec les vecteurs IDLV produits avec les trois plasmides d'expression codant ZsGreenl, mCherry et mtBFP simultanément.

Le pourcentage de cellules fluorescentes a été quantifié par cytométrie en flux.

Des cellules HCT116 (ATCC, CCL-247) sont ensemencées en plaque 96 puits et incubées 24h à 37°C / 5% CO2. La transduction par les particules MS2RLP ou les vecteurs IDLV est réalisée en présence de 4µg/mL Polybrene. A différents temps post-transduction (8h, 24h et 48h), les cellules sont récupérées et le pourcentage de cellules exprimant la ZsGreenl, la mCherry et la mtBFP est quantifié par cytométrie (Macs Quant VYB, Miltenyi Biotec). Un inhibiteur des mécanismes de défense cellulaire, le BX795 (InvivoGen), est utilisé à une concentration de 6µM. Chaque expérience est réalisée en triplicat.

### II. Résultats

### 1. Capacité à transférer plusieurs ARN différents en une seule transduction avec des particules MS2RLP

L'objectif des expériences suivantes est de démontrer la capacité des particules MS2RLP à transférer plusieurs ARN différents au sein de cellules cibles, permettant ainsi de réduire le nombre de transductions sur les cellules cibles. Ainsi, il devient possible de transférer plusieurs ARN différents en une seule transduction plutôt que de devoir réaliser une transduction par espèce d'ARN. Pour cela, nous avons généré des lots purifiés de particules MS2RLP, selon les conditions optimales définies dans l'exemple 1, c'est-à-dire des lots purifiés, produits sans sérum, selon le procédé décrit dans la demande WO 2013/014537. Lors de la phase de production des particules MS2RLP, trois types de plasmides codants pour des rapporteurs fluorescents ont été utilisés simultanément en quantité équimolaire (ZsGreenl, mCherry et mtBFP), avec les plasmides d'encapsidation et d'enveloppe, afin d'obtenir un lot de particules MS2RLP comprenant plusieurs types d'ARN différents. Les cellules HCT116 ont été ensuite transduites en présence de BX795, et le transfert des différents ARN est observé à 8h, 24h et 48h post-transduction.

La Figure XX illustre la proportion de cellules tri-fluorescentes dans le vert, le rouge et le bleu, après une seule transduction de cellules HCT116 par des particules MS2RLP à deux doses différentes (100000 PP/cellule et 300000 PP/cellule), ou après trois transductions simultanées de particules MS2RLP exprimant la ZsGreenl ou la mCherry ou la mtBFP produites selon l'exemple 1, à une dose finale de 300000 PP/cellule.

Contrairement aux résultats de l'exemple 1 montrant qu'il est possible de détecter l'expression d'une seule protéine fluorescente ou luminescente à 8 heures, l'expression des trois fluorescences à 8h post-transduction n'est pas détectable, à une dose de 100000 PP/cellule. En revanche, 48% de cellules sont trifluorescentes à 24h post-transduction. Cette proportion de cellules trifluorescentes augmente, jusqu'à 60% à 48h post-transduction. Les résultats montrent donc que les particules MS2RLP, sont capables de transporter et de transférer au moins 3 types d'ARN en une seule transduction des cellules cibles.

La détermination de la dose optimale de particules par cellule est importante car on observe que l'augmentation de la dose de particules MS2RLP se traduit par une diminution du nombre de cellules trifluorescentes (Figure XX, 300000 PP/cellule) en particulier 48 heures après la transduction. En effet, si la proportion de cellules trifluorescentes à 300000 PP/cellule est un peu plus importante à 8h post-transduction (5% de cellules trifluorescentes) qu'à 100000 PP/cellule, la proportion de cellules trifluorescentes commence à diminuer à 24h post-transduction en passant de 48% à 40% de cellules trifluorescentes. Cet écart d'efficacité de transfert entre les deux doses utilisées est d'autant plus important à 48h post-transduction : en effet, on observe 43% de cellules trifluorescentes pour la dose de 300000 PP/cellule contre 60% de cellules trifluorescentes pour la dose de 100000 PP/cellule.

En parallèle, trois transductions simultanées de cellules HCT116 avec des lots de particules MS2RLP produits individuellement selon l'exemple 1 ont été réalisées. La dose utilisée est de 100000 PP/cellules pour chaque type de fluorescence, soit une dose totale de 300000 PP/cellule. Les résultats montrent que le pourcentage de cellules trifluorescentes obtenu par trois transductions simultanées de particules porteuses de seulement une espèce d'ARN est moins important que celui induit par une seule transduction par des particules porteuses des 3 espèces d'ARN. En effet, seulement 7% de cellules trifluorescentes sont détectés à 8h post-tranduction, puis 20% de cellules trifluorescentes à 24h post-transduction, soit seulement 42% du niveau obtenu avec la transduction unique à 100000 PP/cellule et 50% du niveau obtenu avec la transduction unique à 300000 PP/cellule ; et enfin 23% de cellules trifluorescentes à 48h post-transduction, soit 38% du pourcentage obtenu avec la transduction unique à 100000 PP/cellule et 54% du pourcentage obtenu avec la transduction unique à 300000 PP/cellule. En conclusion, la tri-transduction ne permet pas d'atteindre des pourcentages de cellules tri-colorées aussi élevés que ceux obtenus avec les trois transductions simultanées.

La mise en évidence de la capacité de transfert de différents types d'ARN en une seule transduction d'un même lot de particules MS2RLP représente un gain significatif sur le transfert simultané de ces différents ARN dans les cellules cibles. Cette nouvelle propriété des particules MS2RLP, ayant pour conséquence directe l'optimisation du nombre de transduction à réaliser, permet de ne pas compromettre la viabilité des cellules cibles à la fois par un nombre trop important de transduction ainsi que par la mise en contact des cellules avec une trop grande quantité de particules.

### 2. Comparaison avec un vecteur IDLV

Les mêmes expériences qu'au point 1 ci-dessus ont été reproduites avec un autre type de particules en parallèle: un vecteur viral déficient pour l'intégration par une mutation en position 64 sur la séquence codant l'Intégrase (IDLV).

La Figure XXI illustre la proportion de cellules tri-fluorescentes dans le vert, le rouge et le bleu, après une seule transduction de cellules HCT116 avec des particules MS2RLP produites avec les 3 plasmides d'expression ZsGreenl, mCherry et mtBFP, en comparaison avec trois transductions de cellules HCT116 avec des vecteurs IDLV exprimant la ZsGreenl ou la mCherry ou la mtBFP produites selon l'exemple 1, à une dose totale de 300000 PP/cellule.

Les vecteurs IDLV sont produites dans les mêmes conditions que les particules MS2RLP afin de permettre de réaliser une transduction unique, à la même dose que celle utilisée pour les particules MS2RLP. A 24h après la transduction unique à une dose de 100000 PP/cellule, la proportion de cellules trifluorescentes est légèrement plus importante dans le cas de l'utilisation de vecteurs IDLV par rapport à celle obtenue avec les particules MS2RLP (resp. 56% et 48%). Cette tendance est confirmée 48h après la transduction unique à une dose de 100000 PP/cellule (resp. 80% et 60%).

A 24h post-transduction, la transduction unique des cellules HCT116 par des vecteurs IDLV se traduit par un pourcentage de cellules tri-colorées équivalent à celui obtenu après tri-transduction par des vecteurs IDLV. Cette tendance reste stable à 48h post-transduction, avec un écart minime entre les 2 procédés de transduction (71% pour la tri-transduction contre 81% pour la transduction unique). Donc, contrairement aux résultats obtenus avec les particules MS2RLP, les vecteurs IDLV produits avec 3 plasmides d'expression ne permettent pas une augmentation notable du pourcentage de cellules tri-colorées lors de leur transduction, en comparaison avec une tri-transduction. Cet avantage des particules MS2RLP s'explique vraisemblablement par leur capacité à encapsider plus de molécules d'ARN que les IDLV ou ILV (qui n'encapsident strictement que 2 molécules). Ainsi l'expression de 3 transgènes différents est facilitée car elle ne nécessite pas la transduction d'une même cellule par plusieurs particules lentivirales.

On notera par ailleurs que le pourcentage de cellules trifluorescentes à 8h post-transduction n'est pas détectable avec les vecteurs IDLV à 300000 PP/cellule dans le cas des trois transductions ou dans celui de la monotransduction. A 300000 PP/cellule, les particules MS2RLP permettent en revanche de détecter des cellules trifluorescentes à 8h post-transduction (Figure XX).

Dans le cas des trois mono-transductions par des vecteurs IDLV, la proportion de cellules trifluorescentes est légèrement plus importante à 24h et 48h après les trois transductions (resp. 60% et 70% de cellules trifluorescentes) que dans le cas d'une transduction unique de particules MS2RLP à 100000 PP/cellule (resp. 48% et 60% de cellules trifluorescentes). Il est important de noter que cette différence de transfert est d'environ 20% pour une dose utilisée pour les vecteurs IDLV 3 fois supérieure à la dose optimale des particules MS2RLP.

De plus, les particules IDLV, bien que déficientes pour l'intégration, ne permettent pas de s'affranchir d'intégrations résiduelles dans le génome des cellules cibles (Nightingale et al., 2006 et Apolonia et al., 2007). De ce fait, la probabilité d'événements d'intégration résiduelle augmente avec la dose de particules IDLV utilisée. L'utilisation des particules MS2RLP, par leur nature, permet donc de s'affranchir de cette contrainte par l'absence totale d'intégration résiduelle, quelle que soit la dose utilisée.

### 3. Comparaison avec un vecteur ILV

Les Figures XX et XXII montrent les profils de transduction des particules MS2RLP et des vecteurs ILV, qui sont caractéristiques de l'encapsidation de deux molécules d'ARN viraux, dans des conditions permettant de voir un effet quantitatif de la tri-transduction vs. transduction unique (conditions non saturantes).

### Transduction des cellules HCT116 par des vecteurs ILV, observation à 24h post-transduction (Figure XXII)

La tri-transduction des cellules avec des vecteurs ILV à MOI5 impliquant l'utilisation de lots de vecteurs différents codant chacun pour une protéine fluorescente différente (MOI finale=3^{∗}5=15) permet d'obtenir 7,5% de cellules exprimant les 3 couleurs. En comparaison, la transduction unique à la même dose (MOI15) ne permet d'obtenir que 0,8% de cellules exprimant les 3 couleurs. Le pourcentage de cellules exprimant les 3 couleurs diminue donc entre tri-transduction et transduction unique, la transduction unique n'est donc pas le procédé le plus adapté pour l'utilisation des ILV.

Cette différence se confirme à d'autres doses. La tri-transduction des cellules avec des vecteurs impliquant l'utilisation de lots de vecteurs différents codant chacun pour une protéine fluorescente différente à MOI20 (MOI finale=3^{∗}20=60) permet d'obtenir près de 75% de cellules exprimant les 3 couleurs tandis qu'en transduction unique à MOI 60, seulement 38% de cellules expriment les 3 couleurs.

Même si la dose d'ILV utilisée est augmentée au-delà de la dose optimale, par exemple une MOI120, la transduction unique d'ILV ne permet ni d'atteindre le pourcentage de cellules exprimant les 3 couleurs obtenu en tri-transduction à MO1120 (61,7% vs. 83,3%), ni celui obtenu en tri-transduction à MOI 60 (61,7% vs. 75,6%).

Les MOI utilisées 5, 10, 20, 60 permettent de se placer dans des conditions de transduction non saturantes permettant de mesurer des différences de transductions effectives.

### Transduction des cellules HCT116 par des particules MS2RLP, observation à 24h post-transduction (Figure XX)

La tri-transduction des cellules avec des particules MS2RLP impliquant l'utilisation de lots de vecteurs différents codant chacun pour une protéine fluorescente différente à une dose de 10pg/cellule pour chaque lot de particules MS2RLP (dose finale=3^{∗}10pg=30pg/cellule) permet d'obtenir 20% de cellules exprimant les 3 couleurs. La transduction unique de cellules avec des particules MS2RLP à la même dose (30pg/cellule) permet quant à elle d'obtenir le double de cellules exprimant les 3 couleurs, soit 40%. Le pourcentage de cellules exprimant les 3 couleurs double donc entre tri-transduction et transduction unique, la transduction unique est donc le procédé le plus adapté pour l'utilisation des particules MS2RLP. Ce résultat est inverse à celui obtenu avec des vecteurs ILV.

Les particules MS2RLP montrent donc un profil de transduction différent de celui obtenu par l'utilisation de vecteurs ILV qui s'explique par la différence de mode de formation des vecteurs ou particules. En effet, les particules MS2RLP diffèrent des vecteurs ILV par leur système de recrutement hétérologue d'ARN non viraux. Dans le cas du vecteur lentiviral intégratif ILV, le recrutement par le système d'encapsidation médié par la séquence Psi limite le nombre de molécules d'ARN viral dans la particule à 2. Nos résultats de quantification du nombre de molécules d'ARN dans la particule lentivirale MS2RLP ont estimé qu'en moyenne, 6 molécules d'ARN sont encapsidées dans la particule MS2RLP. Ces encapsidations différentielles peuvent expliquer les différences de transduction par des séquences multiples entre ILV et MS2RLP.

D'autre part, avec les particules MS2RLP en transduction unique à 10pg/cellule, qui est la dose optimale d'utilisation, on obtient 48% de cellules exprimant les 3 couleurs, alors qu'en tri-transduction à une dose 3 fois plus importante (soit 30 pg/cellule), on n'obtient que 20% de cellules exprimant les 3 couleurs, soit 2,5 fois moins de cellules exprimant les 3 couleurs.

En conclusion, ces derniers résultats montrent que seules les particules MS2RLP sont capables d'introduire plus de 2 espèces d'ARN dans des cellules cibles à partir d'une seule composition de particules et ce à des temps courts post-transduction ou injection (5-8 heures) sans induire d'événement d'intégration.

### EXEMPLE 3 : Comparaison de l'efficacité de transduction des cellules HCT116 par des particules MS2RLP-ZsGreen produites selon le procédé P1 ou P2, décrits à l'Exemple 1.

### I. Matériel & Méthodes

Cet exemple est réalisé en utilisant des particules MS2RLP permettant le transfert d'un seul type d'ARN permettant l'expression d'une seule protéine (ZsGreenl).

Les particules ont été produites par transfection de cellules HEK293T avec un seul plasmide d'expression codant la ZsGreenl, (comme cela est décrit à l'Exemple 1), en plus des plasmides d'encapsidation et de l'enveloppe, et en suivant les procédés de production P1 ou P2, décrits à l'Exemple 1.

Des cellules HCT116 (ATCC, CCL-247) sont ensemencées à 25000 cellules/cm² en plaque 96 puits et incubées 24h à 37°C / 5% CO₂.

La transduction par les particules MS2RLP est réalisée en présence de 4µg/mL Polybrene® avec différentes quantités de particules MS2RLP/cellule (10, 20 et 30pg p24/cellule). 48h post-transduction, les cellules sont récupérées et le pourcentage de cellules exprimant la ZsGreenl ainsi que l'intensité de fluorescence sont quantifiés par cytométrie (Macs Quant VYB, Miltenyi Biotec). Un inhibiteur des mécanismes de défense cellulaire, le BX795 (InvivoGen), est utilisé à une concentration de 6µM. Chaque expérience est réalisée en triplicat.

### II. Résultats

L'objectif de cette expérience est de comparer l'efficacité de transduction des cellules HCT116 par des particules MS2RLP-ZsGreen produites selon le procédé P1 ou P2. Les résultats présentés sur la figure XXIII montrent que la capacité de transfert d'ARN n'est pas impactée par le procédé de production car le pourcentage de cellules transduites est le même quelque soit le procédé de production utilisé. En revanche, seule l'expression du transgène est impactée par le procédé de production. En effet, les particules purifiées permettent d'obtenir le niveau d'expression de la protéine d'intérêt le plus élevé, quelque soit la dose de particules MS2RLP utilisées.

### EXEMPLE 4 : Construction de particules lentivirales MS2RLP par modification de la nucléocapside et test du nombre de répétition du motif tige boucle de l'ARN MS2

### I. Matériel & Méthodes

### 1. Constructions des plasmides

- **Plasmide d'expression d'une séquence d'intérêt** : Le plasmide d'expression est porteur d'une cassette d'expression promoteur-séquence d'intérêt-polyA (voir Figure II) avec ou non une séquence intronique ou stabilisatrice d'ARN. Afin de véhiculer les ARNm dans les particules lentivirales, plusieurs répétions du motif tige-boucle de l'ARN MS2 (ctagaaaacatgaggatcacccatgtctgcag, SEQ ID N°1) ont été insérées au sein d'une cassette d'expression en aval du gène rapporteur :
   - 2 répétitions (Figure XXIV)
   - 6 répétions (Figure XXV)
   - 12 répétitions (Figure XXVI)
   La séquence d'intérêt choisie est celle de la Luciferase Firefly native.
- **Plasmide d'encapsidation** : Le plasmide d'encapsidation est celui décrit à l'Exemple 1 (Figure IIIb).
- **Plasmide de l'enveloppe (pENV)** : Le plasmide d'encapsidation est celui décrit à l'Exemple 1 (Figure IV).

### 2. Production, concentration/purification et titration des particules lentivirales

Les particules lentivirales sont produites comme décrit à l'Exemple 1 et elles sont concentrées et purifiées selon le procédé P1 tel que décrit à l'Exemple 1.

### 3. Cinétique d'expression de la Luciférase

Les cellules HCT116 (ATCC, CCL-247) sont ensemencées en plaque 96 puits et incubées 24h à 37°C / 5% CO₂. La transduction par les particules MS2RLP2X, 6X, 12X est réalisée à une dose de 100 000 PP/cellule (10pg p24/cellule), en présence de 4µg/mL Polybrene®. Le surnageant de transduction est éliminé 4 heures après et remplacé par du milieu de culture supplémenté frais. A 4h, 8h, 24h, 32h, et 48h post-transduction, les cellules sont récupérées et l'expression de la Luciérase est analysée à l'aide du kit OneGlo Luciferase assay (Promega) en suivant les recommandations du fournisseur et en utilisant le lecteur de plaque Synergy H1 Hybrid (Biotek). Ce test est réalisé en triplicat. Le contrôle est réalisé par des cellules HCT116 non transduites.

### II. Résultats

Les résultats sont présentés sur la Figure XXVII. L'objectif de cette expérience est de vérifier qu'il est possible de diminuer le nombre de motifs répétés de la séquence MS2 sur le plasmide d'expression sans que le niveau d'expression de l'ARN à délivrer ne soit impacté. La cinétique d'expression de la Luciferase présente une augmentation du signal de luminescence de 4h à 8h, montrant que dans le cas d'une protéine avec une durée de vie courte, le maximum d'expression est atteint précocement, quelque soit le nombre de motifs répétés de la séquence MS2. Après 8h, l'activité de la Luciferase diminue, jusqu'à atteindre à 48h le même niveau qu'à 4h post-transduction (entre 10 000 et 20 000 URL). Les constructions portant 2 et 6 motifs répétés de la séquence MS2 montrent le même profil d'expression de la Luciférase au cours du temps. En revanche, l'expression de la Luciférase par la construction portant 12 motifs répétés de la séquence MS2 est 30% plus forte que les constructions portant 2 ou 6 motifs répétés. Par conséquent, les particules MS2RLP sont efficaces pour délivrer des ARN quelque soit le nombre de motifs répétés utilisé. Selon l'application, le nombre de répétitions du motif MS2 devra être adapté.

### EXEMPLE 5 : Construction de particules lentivirales MS2RLP par modification de l'intégrase et test du nombre de répétition du motif tige boucle de l'ARN MS2

### I. Matériel & Méthodes

### 1. Constructions des plasmides

- **Plasmide d'expression d'une séquence d'intérêt** : Le plasmide d'expression est porteur d'une cassette d'expression promoteur-séquence d'intérêt-polyA (voir Figure II) avec ou non une séquence intronique ou stabilisatrice d'ARN. Afin de véhiculer les ARNm dans les particules lentivirales, plusieurs répétions du motif tige-boucle de l'ARN MS2 (ctagaaaacatgaggatcacccatgtctgcag, SEQ ID N°1) ont été insérées au sein d'une cassette d'expression en aval du gène rapporteur :
   - 2 répétitions (Figure XXIV)
   - 6 répétions (Figures XXV)
   - 12 répétitions (Figure XXVI)

   Le promoteur utilisé peut être celui du CMV (Figure IIa) ou EF1 (Figure IIb) mais d'autres promoteurs peuvent être utilisés. La séquence d'intérêt peut être un ADN codant une protéine reportrice comme la Luciferase Firefly native (Figure IIa), une protéine fluorescente (Figure IIb) verte (ZsGreenl), rouge (mCherry) ou bleue (mtBFP), ou un ADNc codant une protéine, par exemple la protéine CRE (Figure IIc). La séquence d'intérêt peut également être celle d'un shRNA, d'un miRNA, d'un sgRNA, d'un LncRNA ou d'un circRNA.
- **Plasmide d'encapsidation** : La particule lentivirale a été modifiée pour contenir au sein de l'intégrase, la séquence de la protéine « Coat » du bactériophage MS2. Le plasmide d'encapsidation p8.74 (Figure VI), porteur des gènes codant les protéines de structures et de fonction (Gag, Pol), utilisé pour la production des particules MS2RLP est modifié selon la stratégie illustrée par la Figure I : ce plasmide p8.74 est utilisé pour générer, par PCR d'assemblage, un plasmide sur lequel la protéine Coat du phage MS2 est fusionnée au domaine C terminal de l'intégrase. Cette fusion, obtenue par clonage Hpal, permet de générer le plasmide p8.74-POL-MS2 Coat Linéaire (Figure XXVIII). On obtient ainsi une construction telle qu'illustrée en Figure I (p8.74.IN-coat). La séquence codant Pol peut être délétée ou mutée dans certains éléments de fonction comme par exemple la séquence codant la transcriptase inverse (RT).
- **Plasmide de l'enveloppe (pENV)** : Ce plasmide est porteur du gène codant une protéine d'enveloppe, qui peut être la VSVG codant la protéine d'enveloppe du Virus de la stomatite vésiculaire (Figure IV).

### 2. Production, concentration/purification et titration des particules lentivirales

Les particules lentivirales sont produites comme décrit à l'Exemple 1, selon le procédé P1.

### 3. Cinétique d'expression de la Luciférase

Les cellules HCT116 (ATCC, CCL-247) sont ensemencées en plaque 96 puits et incubées 24h à 37°C / 5% CO₂. La transduction par les particules MS2(IN)-RLP2X, 6X, 12X produites selon le procédé P1 est réalisée à une dose de 5pg p24/cellule, en présence de 8µg/mL Polybrene®. Le surnageant de transduction est éliminé 4 heures après et remplacé par du milieu de culture supplémenté frais. A 4h, 8h, 24h, 32h, et 48h post-transduction, les cellules sont récupérées et l'expression de la Luciférase est analysée à l'aide du kit OneGlo Luciferase assay (Promega) en suivant les recommandations du fournisseur et en utilisant le lecteur de plaque Synergy H1 Hybrid (Biotek). Ce test est réalisé en triplicat. Le contrôle est réalisé par des cellules HCT116 non transduites.

### II. Résultats

Les résultats sont présentés sur la figure XXIX. L'objectif de cette expérience est de vérifier qu'il est possible de véhiculer des ARN dans les particules lentivirales avec MS2-Coat dans l'intégrase et de diminuer le nombre de motifs répétés de la séquence MS2 sur le plasmide d'expression sans que le niveau d'expression de l'ARN à délivrer ne soit impacté. Le maximum d'expression de la Luciférase est atteint précocement, quelque soit le nombre de motifs répétés de la séquence MS2. Le signal de luminescence le plus fort de la cinétique d'expression de la Luciférase est obtenu dès 4h et jusqu'à 24h. Après 24h, l'activité de la Luciférase diminue jusqu'à atteindre un signal 2 à 4 fois plus faible que pour les conditions à 4/8/24h. Les particules MS2(IN)-RLP permettent donc de délivrer des ARN, quelque soit le nombre de motifs répétés de la séquence MS2.

### EXEMPLE 6 : Construction de particules lentivirales PP7(NC)-RLP et test de l'effet dose des particules PP7RLP sur la transduction de cellules HCT116

### I. Matériel & Méthodes

### 1. Constructions des plasmides

### 1.1 Plasmides pour la production de particules lentivirales PP7(NC)-RLP Luc 12X

- **Plasmide d'expression d'une séquence d'intérêt** : Le plasmide d'expression est porteur d'une cassette d'expression promoteur-séquence d'intérêt-polyA (voir Figure XXX) avec ou non une séquence intronique ou stabilisatrice d'ARN. Afin de véhiculer les ARNm dans les particules lentivirales, 12 répétions du motif tige-boucle de l'ARN PP7 (ctagaaggagcagacgatatggcgtcgctccctgcag SEQ ID N°5) ont été insérées au sein d'une cassette d'expression en aval du gène rapporteur.
   Le promoteur utilisé peut être celui du CMV ou EF1 (Figure XXX) mais d'autres promoteurs peuvent être utilisés. La séquence d'intérêt peut être un ADN codant une protéine reportrice comme la Luciferase Firefly native (Figure IIa), une protéine fluorescente (Figure IIb) verte (ZsGreenl), rouge (mCherry) ou bleue (mtBFP), ou un ADNc codant une protéine, par exemple la protéine CRE (Figure IIc). La séquence d'intérêt peut également être celle d'un shRNA, d'un miRNA, d'un sgRNA, d'un LncRNA ou d'un circRNA.
- **Plasmide d'encapsidation** : La particule lentivirale a été modifiée pour contenir au sein de la protéine de nucléocapside, en lieu et place du second domaine en doigt de Zn, la séquence de la protéine « Coat » du bactériophage PP7. Le plasmide d'encapsidation p8.74, porteur des gènes codant les protéines de structures et de fonction (Gag, Pol), utilisé pour la production des particules PP7RLP est modifié selon la stratégie illustrée par la Figure XXXI : ce plasmide p8.74 est utilisé pour générer, par PCR d'assemblage, un plasmide dépourvu du second doigt de zinc de la protéine de nucléocapside p8.74ΔZF. Le second doigt de zinc est substitué par la protéine Coat du phage PP7 par clonage Hpal, pour générer le plasmide p8.74ΔZF-PP7-Coat. On obtient ainsi la construction illustrée en Figure XXXII. La séquence codant Pol peut être délétée ou mutée dans certains éléments de fonction comme par exemple la séquence codant la transcriptase inverse (RT) ou l'intégrase (IN) sans altérer la fonction des PP7RLP.
- **Plasmide de l'enveloppe (pENV)** : Ce plasmide est porteur du gène codant une protéine d'enveloppe, qui peut être la VSVG codant la protéine d'enveloppe du Virus de la stomatite vésiculaire (Figure IV).

### 1.2 Plasmides pour la production de particules lentivirales MS2(NC)-RLP Luc 12X

Les plasmides utilisés sont identiques à ceux utilisés dans l'Exemple 1.

### 2. Production, concentration/purification et titration des particules lentivirales

Les particules lentivirales sont produites comme décrit à l'Exemple 1, selon le procédé P1.

### 3. Effet dose des particules PP7(NC)-RLP-Luc

Les cellules HCT116 (ATCC, CCL-247) sont ensemencées en plaque 96 puits et incubées 24h à 37°C / 5% CO₂. La transduction par les particules PP7(NC)-RLP-Luc-12X, produites selon le procédé P1 est réalisée selon une dose allant de 0 à 20pg p24/cellule, en présence de 8µg/mL Polybrene®. 4h post-transduction, les cellules sont récupérées et l'expression de la Luciférase est analysée à l'aide du kit OneGlo Luciferase assay (Promega) en suivant les recommandations du fournisseur et en utilisant le lecteur de plaque Synergy H1 Hybrid (Biotek). Ce test est réalisé en triplicat. Le contrôle est réalisé par des cellules HCT116 non transduites.

### II. Résultats

Les résultats sont présentés sur la figure XXXIII. L'objectif de cette expérience est dans un premier temps, de voir si le système d'interaction ARN/protéine du bactériophage PP7 permet l'encapsidation d'ARN lors de la formation des particules RLP. Dans un second temps, cette expérience permet de déterminer les meilleures conditions expérimentales d'utilisation des particules PP7(NC)-RLP.

La figure XXXIII montre que dès la dose de 1pg p24/cellule, l'activité Luciférase est fortement détectée. Plus on augmente la dose de PP7(NC)-RLP, et plus l'activité Luciférase est forte. De plus, en comparant avec le vecteur MS2(NC)-RLP utilisé à la même dose que le PP7(NC)-RLP, le signal de l'activité Luciférase est très fortement comparable.

Cette expérience montre qu'avec 12 motifs répétés de la séquence PP7, le système d'encapsidation des ARN par la protéine Coat du bactériophage PP7 fonctionne aussi bien que le système issu du bactériophage MS2, et que pour obtenir une très forte expression de la protéine d'intérêt, on peut se placer à une dose de 10pg p24/cellule.

### EXEMPLE 7 : Construction de particules lentivirales PP7(NC)-RLP et test du nombre de répétitions du motif tige boucle de l'ARN PP7

### I. Matériel & Méthodes

### 1. Constructions des plasmides

### 1.1 Plasmides pour la production de particules lentivirales PP7(NC)-RLP

- **Plasmide d'expression d'une séquence d'intérêt** : Le plasmide d'expression est porteur d'une cassette d'expression promoteur-séquence d'intérêt-polyA (voir Figure XXX) avec ou non une séquence intronique ou stabilisatrice d'ARN. Afin de véhiculer les ARNm dans les particules lentivirales, plusieurs répétions du motif tige-boucle de l'ARN PP7 (ctagaaggagcagacgatatggcgtcgctccctgcag SEQ ID N°5) ont été insérées au sein d'une cassette d'expression en aval du gène rapporteur :
   - 2 répétitions (Figure XXXIVa)
   - 6 répétions (Figures XXXV)
   - 12 répétitions (Figure XXX)

   Le promoteur utilisé peut être celui du CMV ou EF1 (Figure XXX) mais d'autres promoteurs peuvent être utilisés. La séquence d'intérêt peut être un ADN codant une protéine reportrice comme la Luciferase Firefly native (Figure XXXIVa), une protéine fluorescente (Figure XXXIVb) verte (ZsGreenl), rouge (mCherry) ou bleue (mtBFP), ou un ADNc codant une protéine, par exemple la protéine CRE. La séquence d'intérêt peut également être celle d'un shRNA, d'un miRNA, d'un sgRNA, d'un LncRNA ou d'un circRNA.
- **Plasmide d'encapsidation** : La particule lentivirale a été modifiée pour contenir au sein de la protéine de nucléocapside, en lieu et place du second domaine en doigt de Zn, la séquence de la protéine « Coat » du bactériophage PP7. Le plasmide d'encapsidation p8.74, porteur des gènes codant les protéines de structures et de fonction (Gag, Pol), utilisé pour la production des particules PP7RLP est modifié selon la stratégie illustrée par la Figure XXXI : ce plasmide p8.74 est utilisé pour générer, par PCR d'assemblage, un plasmide dépourvu du second doigt de zinc de la protéine de nucléocapside p8.74ΔZF. Le second doigt de zinc est substitué par la protéine Coat du phage PP7 par clonage Hpal, pour générer le plasmide p8.74ΔZF-PP7-Coat. On obtient ainsi la construction illustrée en Figure XXXII. La séquence codant Pol peut être délétée ou mutée dans certains éléments de fonction comme par exemple la séquence codant la transcriptase inverse (RT) ou l'intégrase (IN) sans altérer la fonction des PP7RLP.
- **Plasmide de l'enveloppe (pENV)** : Ce plasmide est porteur du gène codant une protéine d'enveloppe, qui peut être la VSVG codant la protéine d'enveloppe du Virus de la stomatite vésiculaire (Figure IV).

### 1.2 Plasmides pour la production de particules lentivirales MS2(NC)-RLP Luc 12X

Les plasmides utilisés sont identiques à ceux utilisés dans l'Exemple 1.

### 2. Production, concentration/purification et titration des particules lentivirales

Les particules lentivirales sont produites comme décrit à l'Exemple 1, selon le procédé P1.

### 3. Cinétique d'expression de la Luciferase

Les cellules HCT116 (ATCC, CCL-247) sont ensemencées en plaque 96 puits et incubées 24h à 37°C / 5% CO₂. La transduction par les particules PP7(NC)-RLP-Luc-2X, 6X, 12X produites selon le procédé P1 est réalisée à une dose de 10pg p24/cellule, en présence de 8µg/mL Polybrene®. Le surnageant de transduction est éliminé 4 heures après et remplacé par du milieu de culture supplémenté frais. A 4h, 8h, 24, 32, et 48h post-transduction, les cellules sont récupérées et l'expression de la Luciferase est analysée à l'aide du kit OneGIo Luciferase assay (Promega) en suivant les recommandations du fournisseur et en utilisant le lecteur de plaque Synergy H1 Hybrid (Biotek). Ce test est réalisé en triplicat. Le contrôle est réalisé par des cellules HCT116 non transduites.

### II. Résultats

Les résultats sont présentés sur la figure XXXVI. L'objectif de cette expérience est de montrer l'impact des modifications du nombre de motifs répétés de la séquence PP7 sur la capacité d'encapsidation et donc sur le transfert d'ARN dans des cellules cibles, à plusieurs temps donnés. Les particules PP7(NC)-RLP comprenant le motif PP7 répété 2 fois, 6 fois ou 12 fois permettent le transfert d'ARN dans des proportions comparables à celles obtenues avec des particules MS2(NC)-RLP comprenant le motif MS2 12 fois. Les particules PP7(NC)-RLP sont donc efficaces pour délivrer des ARN quelque soit le nombre de motifs répétés utilisé. Selon le type d'application, le nombre de répétitions pourra donc être adapté. Les résultats montrent que, dans les conditions de l'expérience, les particules PP7(NC)-RLP comprenant le motif PP7 répété 2 fois ou 6 fois permettent le transfert d'ARN plus efficace que celui obtenu par l'utilisation de particules MS2(NC)-RLP comprenant le motif de MS2 12 fois. La cinétique d'expression de la Luciferase présente une augmentation du signal de luminescence de 4h à 8h, montrant que dans le cas d'une protéine avec une durée de vie courte, le maximum d'expression est atteint précocement, quelque soit le nombre de motifs répétés de la séquence PP7. Après 24h, l'activité de la Luciferase diminue, jusqu'à atteindre à 32h et 48h un signal de luminescence 4 à 5 fois moins intense que pour la condition 8h post-transduction.

### EXEMPLE 8 : Construction de particules lentivirales PP7(IN)-RLP par modification de l'intégrase et test du nombre de répétition du motif tige boucle de l'ARN PP7

### I. Matériel & Méthodes

### 1. Constructions des plasmides

- **Plasmide d'expression d'une séquence d'intérêt** : Le plasmide d'expression est porteur d'une cassette d'expression promoteur-séquence d'intérêt-polyA (voir Figure XXX) avec ou non une séquence intronique ou stabilisatrice d'ARN. Afin de véhiculer les ARNm dans les particules lentivirales, plusieurs répétions du motif tige-boucle de l'ARN PP7 (ctagaaggagcagacgatatggcgtcgctccctgcag SEQ ID N°5) ont été insérées au sein d'une cassette d'expression en aval du gène rapporteur :
   - 2 répétitions (Figure XXXIVa)
   - 6 répétions (Figures XXXV)
   - 12 répétitions (Figure XXX)

   Le promoteur utilisé peut être celui du CMV ou EF1 (Figure XXX) mais d'autres promoteurs peuvent être utilisés. La séquence d'intérêt peut être un ADN codant une protéine reportrice comme la Luciferase Firefly native (Figure XXXIVa), une protéine fluorescente (Figure XXXIVb) verte (ZsGreenl), rouge (mCherry) ou bleue (mtBFP), ou un ADNc codant une protéine, par exemple la protéine CRE (Figure XXXIVc). La séquence d'intérêt peut également être celle d'un shRNA, d'un miRNA, d'un sgRNA, d'un LncRNA ou d'un circRNA.
- **Plasmide d'encapsidation** : La particule lentivirale a été modifiée pour contenir au sein de l'intégrase, la séquence de la protéine « Coat » du bactériophage PP7. Le plasmide d'encapsidation p8.74, porteur des gènes codant les protéines de structures et de fonction (Gag, Pol), utilisé pour la production des particules PP7(IN)-RLP est modifié selon la stratégie illustrée par la Figure XXXVII : ce plasmide p8.74 est utilisé pour générer, par PCR d'assemblage, un plasmide sur lequel la protéine Coat du phage PP7 est fusionnée au domaine C terminal de l'intégrase. Cette fusion, obtenue par clonage Hpal, permet de générer le plasmide P8.74- POL-PP7 Coat. On obtient ainsi la construction illustrée en Figure XXXVIII. La séquence codant Pol peut être délétée ou mutée dans certains éléments de fonction comme par exemple la séquence codant la transcriptase inverse (RT).
- **Plasmide de l'enveloppe (pENV)** : Ce plasmide est porteur du gène codant une protéine d'enveloppe, qui peut être la VSVG codant la protéine d'enveloppe du Virus de la stomatite vésiculaire (Figure IV).

### 2. Production, concentration/purification et titration des particules lentivirales

Les particules lentivirales sont produites comme décrit à l'Exemple 1, selon le procédé P1.

### 3. Cinétique d'expression de la Luciferase

Les cellules HCT116 (ATCC, CCL-247) sont ensemencées en plaque 96 puits et incubées 24h à 37°C / 5% CO2. La transduction par les particules PP7(IN)-RLP-Luc 2X, 6X, 12X produites selon le procédé P1 est réalisée à une dose de 2,8 pg p24/cellule, en présence de 8µg/mL Polybrene. Le surnageant de transduction est éliminé 4 heures après et remplacé par du milieu de culture supplémenté frais. A 4h, 8h, 24, 32, et 48h post-transduction, les cellules sont récupérées et l'expression de la Luciferase est analysée à l'aide du kit OneGlo Luciferase assay (Promega) en suivant les recommandations du fournisseur et en utilisant le lecteur de plaque Synergy H1 Hybrid (Biotek). Ce test est réalisé en triplicat. Le contrôle est réalisé par des cellules HCT116 non transduites.

### II. Résultats

Les résultats sont présentés sur la figure XXXIX. L'objectif de cette expérience est de vérifier qu'il est possible de véhiculer des ARN dans les particules lentivirales avec PP7-Coat dans l'intégrase et de montrer l'impact des modifications du nombre de motifs répétés de la séquence PP7 sur la capacité d'encapsidation et donc sur le transfert d'ARN dans des cellules cibles, à plusieurs temps donnés. Le maximum d'expression de la Luciferase est atteint à 8h, quelque soit le nombre de motifs répétés de la séquence PP7. Après 8h, l'activité de la Luciferase diminue, quelque soit le nombre de motifs répétés de la séquence PP7, et la différence d'activité de la Luciferase entre les différents nombres de motifs répétés n'est pas statistiquement significative. Les particules PP7(IN)-RLP permettent donc de délivrer des ARN.

### EXEMPLE 9 : Transfert de plusieurs ARN différents par transduction unique avec des particules PP7RLP

### I. Matériel & Méthodes

### 1. Mono- et tri-transduction

Cet exemple est réalisé en utilisant des particules PP7RLP ou des vecteurs ILV permettant soit le transfert d'un seul type d'ARN permettant l'expression d'une seule protéine (ZsGreenl, mCherry ou mtBFP), soit le transfert de plusieurs types d'ARNs permettant l'expression de plusieurs protéines différentes (ZsGreenl + mCherry + mtBFP).

Les particules PP7RLP et les vecteurs lentiviraux intégratifs ILV sont produits dans les conditions suivantes :
- Les particules ont été produites par transfection de cellules HEK293T avec un seul plasmide d'expression codant soit la ZsGreenl, soit la mCherry, soit la mtBFP (comme cela est décrit à l'Exemple 1), en plus des plasmides d'encapsidation et de l'enveloppe,
- Les particules ont été produites par transfection de cellules HEK293T avec trois plasmides d'expression codant respectivement la ZsGreenl, la mCherry et la mtBFP ajoutés en quantité équimolaire, pour une quantité totale de plasmides d'expression équivalente à la quantité totale de plasmides d'expression utilisée pour une production de particule avec un seul plasmide d'expression, en plus des plasmides d'encapsidation et de l'enveloppe.

Les cellules HCT116 ont été transduites par les particules PP7RLP soit :
- par transduction avec les 3 types de particules PP7RLP ZsGreenl, mCherry ou mtBFP indépendamment, ou par co-transduction des 3 types de particules PP7RLP en même temps
- par mono-transduction avec les particules produites avec les trois plasmides d'expression codant ZsGreenl, mCherry et mtBFP simultanément.

En parallèle, des cellules HCT116 sont transduites par des vecteurs ILV soit :
- par transduction avec les 3 types de vecteurs ILV ZsGreenl, mCherry ou mtBFP indépendamment, ou par co-transduction des 3 types de particules ILV en même temps
- par mono-transduction avec les vecteurs ILV produits avec les trois plasmides d'expression codant ZsGreenl, mCherry et mtBFP simultanément.

Le pourcentage de cellules fluorescentes a été quantifié par cytométrie en flux.

Des cellules HCT116 (ATCC, CCL-247) sont ensemencées en plaque 24 puits et incubées 24h à 37°C / 5% CO₂. La transduction par les particules PP7RLP ou les vecteurs ILV est réalisée en présence de 8µg/mL Polybrene®. 24h post-transduction, les cellules sont récupérées et le pourcentage de cellules exprimant la ZsGreenl, la mCherry et la mtBFP est quantifié par cytométrie (Macs Quant VYB, Miltenyi Biotec). Un inhibiteur des mécanismes de défense cellulaire, le BX795 (InvivoGen), est utilisé à une concentration de 6µM. Chaque expérience est réalisée en triplicat.

### II. Résultats

### 1. Capacité à transférer plusieurs ARN différents en une seule transduction avec des particules PP7RLP et comparaison avec l'ILV

L'objectif des expériences suivantes est de démontrer la capacité des particules PP7RLP à transférer plusieurs ARN différents au sein de cellules cibles, permettant ainsi de réduire le nombre de transductions sur les cellules cibles. Ainsi, il devient possible de transférer plusieurs ARN différents en une seule transduction plutôt que de devoir réaliser une transduction par espèce d'ARN. Pour cela, des lots purifiés de particules PP7RLP ont été générés selon les conditions optimales définies dans l'Exemple 1, c'est-à-dire des lots purifiés, produits sans sérum, selon le procédé P1 de l'Exemple 1 décrit dans la demande WO 2013/014537. Lors de la phase de production des particules PP7RLP, trois types de plasmides codants pour des rapporteurs fluorescents ont été utilisés simultanément en quantité équimolaire (ZsGreenl, mCherry et mtBFP), avec les plasmides d'encapsidation et d'enveloppe, afin d'obtenir un lot de particules PP7RLP comprenant plusieurs types d'ARN différents. Les cellules HCT116 ont été ensuite transduites en présence de BX795, et le transfert des différents ARN est observé 24h post-transduction.

La Figure XL illustre la proportion de cellules tri-fluorescentes dans le vert, le rouge et le bleu, après une seule transduction de cellules HCT116 par des particules PP7RLP à deux doses différentes (10pg p24/cellule et 30pg p24/cellule), ou après trois transductions simultanées de particules PP7RLP exprimant la ZsGreenl ou la mCherry ou la mtBFP produites selon l'Exemple 1, à une dose finale de 30pg p24/cellule.

88% des cellules sont tri-fluorescentes lorsqu'elles sont transduites à une dose de 30pg p24/cellule avec le lot PP7RLP contenant des particules porteuses des 3 espèces d'ARN, alors que seulement 1% des cellules sont fluorescentes lorsqu'elles sont transduites par le lot de vecteurs ILV obtenu par co-transfection des 3 plasmides contenant chacun une protéine fluorescente différente.

Les résultats montrent donc que les particules PP7RLP, sont capables de transporter et de transférer au moins 3 types d'ARN en une seule transduction des cellules cibles. Ces particules permettent le transfert d'ARN multiples beaucoup plus efficacement qu'avec le vecteur ILV.

La transduction de cellules par des ARN multiples est donc beaucoup plus efficace et plus simple avec les particules PP7RLP qu'avec l'ILV, pour un résultat plus important d'au moins 70%.

91% des cellules sont tri-fluorescentes lorsqu'elles sont transduites simultanément à une dose totale de 30pg p24/cellule avec les 3 lots séparés de PP7RLP contenant chacun une seule espèce d'ARN. La comparaison de l'efficacité de l'expression simultanée de plusieurs transgènes en mono-transduction ou multi-transductions montre une efficacité équivalente pour le vecteur PP7RLP (88% vs 91% respectivement).

Cette efficacité permet donc de réduire la multiplicité d'infection par rapport à l'utilisation de lots de particules produits indépendamment. Le risque de toxicité cellulaire en est considérablement réduit. Cela permet de ne pas compromettre la viabilité des cellules cibles à la fois par un nombre trop important de transduction ainsi que par la mise en contact des cellules avec une trop grande quantité de particules.

## Revendications

1. Particule rétrovirale comportant une protéine issue de la polyprotéine Gag, une protéine d'enveloppe, optionnellement une intégrase et au moins deux ARN non viraux différents encapsidés, les ARN non viraux différents encapsidés comportant chacun une séquence d'ARN d'intérêt liée à une séquence d'encapsidation, chaque séquence d'encapsidation étant reconnue par un domaine de liaison introduit dans la protéine issue de la polyprotéine Gag.

2. Particule rétrovirale selon la revendication 1, comportant une protéine de nucléocapside, une protéine d'enveloppe, optionnellement une intégrase et au moins deux ARN non viraux différents encapsidés, les ARN non viraux différents encapsidés comportant chacun une séquence d'ARN d'intérêt liée à une séquence d'encapsidation, chaque séquence d'encapsidation étant reconnue par un domaine de liaison introduit dans la protéine de nucléocapside.

3. Particule rétrovirale selon l'une quelconque des revendications 1 à 2, laquelle est une particule lentivirale.

4. Particule lentivirale selon la revendication 3, dans laquelle :
- le domaine de liaison est la séquence de la protéine « Coat » du bactériophage MS2,
- la séquence d'encapsidation des ARN non viraux est une séquence tige-boucle de MS2,
- la protéine de nucléocapside est la protéine de nucléocapside (NC) de HIV appartenant à la polyprotéine Gag, laquelle NC est mutée au niveau du second doigt de zinc afin d'insérer la séquence de la protéine « Coat » du bactériophage MS2.

5. Particule lentivirale selon la revendication 3, dans laquelle :
- le domaine de liaison est la séquence de la protéine « Coat » du phage PP7,
- la séquence d'encapsidation des ARN non viraux est une séquence tige-boucle de PP7,
- la protéine de nucléocapside est la protéine de nucléocapside (NC) de HIV appartenant à la polyprotéine Gag, laquelle NC est mutée au niveau du second doigt de zinc afin d'insérer la séquence de la protéine « Coat » du bactériophage PP7.

6. Composition comportant des particules selon l'une quelconque des revendications 1 à 5.

7. Kit de production de particules rétrovirales selon la revendication 2, 4 ou 5, comportant :
(i) un plasmide d'expression codant au moins deux séquences d'ARN non virales différentes, chaque séquence d'ARN comportant une séquence d'intérêt pour laquelle en amont ou en aval de cette séquence d'intérêt est insérée une séquence d'encapsidation, ou alternativement un premier et un second plasmide d'expression codant chacun une séquence d'ARN non viral différente comportant une séquence d'intérêt et en amont ou en aval de ladite séquence d'intérêt est insérée une séquence d'encapsidation,
(ii) un plasmide d'encapsidation codant pour une protéine de nucléocapside chimérique comportant un domaine de liaison permettant de reconnaître la séquence d'encapsidation, et,
(iii) un plasmide d'enveloppe codant pour une protéine d'enveloppe.

8. Kit de production de particules rétrovirales selon la revendication 7, dans lequel les séquences d'intérêt sont différentes et les séquences d'encapsidation sont identiques.

9. Procédé de fabrication d'un kit de production de particules rétrovirales selon la revendication 7, comportant :
(i) la préparation d'un plasmide d'expression codant au moins deux séquences d'ARN non virales différentes, chaque séquence d'ARN comportant une séquence d'intérêt pour laquelle en amont ou en aval de cette séquence d'intérêt est insérée une séquence d'encapsidation, ou alternativement un premier et un second plasmide d'expression codant chacun une séquence d'ARN non viral différente comportant une séquence d'intérêt et dans laquelle en amont ou en aval de la séquence d'intérêt est insérée une séquence d'encapsidation,
(ii) la préparation d'un plasmide d'encapsidation codant pour une protéine de nucléocapside chimérique comportant un domaine de liaison permettant de reconnaître la séquence d'encapsidation, et,
(iii) la préparation d'un plasmide d'enveloppe codant pour une protéine d'enveloppe.

10. Procédé de fabrication selon la revendication 9, d'un kit de production de particules rétrovirales selon la revendication 8, dans lequel les séquences d'intérêt sont différentes et les séquences d'encapsidation sont identiques.

11. Procédé de fabrication des particules rétrovirales selon la revendication 2, 4 ou 5, comportant une étape de co-transfection de cellules avec :
(i) un plasmide d'expression codant au moins deux séquences d'ARN non virales différentes, chaque séquence d'ARN comportant une séquence d'intérêt pour laquelle en amont ou en aval de cette séquence d'intérêt est insérée une séquence d'encapsidation, ou alternativement un premier et un second plasmide d'expression codant chacun une séquence d'ARN non viral différente comportant une séquence d'intérêt et dans laquelle en amont ou en aval de la séquence d'intérêt est insérée une séquence d'encapsidation,
(ii) un plasmide d'encapsidation codant pour une protéine de nucléocapside chimérique comportant un domaine de liaison permettant de reconnaître la séquence d'encapsidation, et,
(iii) un plasmide d'enveloppe codant pour une protéine d'enveloppe,
et la récupération du surnageant des cellules transfectées comportant les particules.

12. Procédé de fabrication des particules rétrovirales selon la revendication 11, dans lequel les séquences d'intérêt sont différentes et les séquences d'encapsidation sont identiques.

13. Procédé de fabrication selon la revendication 11 ou 12, dans lequel le surnageant est clarifié.

14. Procédé de fabrication selon la revendication 13, dans lequel le surnageant est en outre concentré et/ou purifié.

15. Utilisation d'une particule selon l'une quelconque des revendications 1 à 5, ou d'une composition selon la revendication 6 pour transduire des cellules *in vitro* ou *ex vivo.*

16. Particule rétrovirale selon l'une quelconque des revendications 1 à 5 ou composition selon la revendication 6, pour son utilisation en thérapie génique.

## Patentansprüche

1. Retrovirales Partikel, das ein vom Polyprotein Gag stammendes Protein, ein Hüllprotein, optional eine Integrase und mindestens zwei verschiedene, kapsidierte, nicht-virale RNS umfasst, wobei die verschiedenen, kapsidierten, nicht-viralen RNS jeweils eine Sequenz von RNS von Interesse umfassen, die mit einer Kapsidationssequenz verbunden ist, wobei jede Kapsidationssequenz durch eine Bindungsdomäne erkannt wird, die in das vom Polyprotein Gag stammende Protein eingebracht wurde.

2. Retrovirales Partikel nach Anspruch 1, umfassend ein Nukleokapsidprotein, ein Hüllprotein, optional eine Integrase und mindestens zwei verschiedene, kapsidierte, nicht-virale RNS, wobei die verschiedenen, kapsidierten, nicht-viralen RNS jeweils eine Sequenz von RNS von Interesse umfassen, die mit einer Kapsidationssequenz verbunden ist, wobei jede Kapsidationssequenz durch eine Bindungsdomäne erkannt wird, die in das Nukleokapsidprotein eingebracht wurde.

3. Retrovirales Partikel nach einem der Ansprüche 1 bis 2, das ein lentivirales Partikel ist.

4. Lentivirales Partikel nach Anspruch 3, wobei:
- die Bindungsdomäne die Sequenz des Proteins « Coat » des Bakteriophagen MS2 ist,
- die Kapsidationssequenz der nicht-viralen RNS eine Haarnadelstruktursequenz von MS2 ist,
- das Nukleokapsidprotein das Protein von Nukleokapsid (NC) von HIV ist, das zum Polyprotein Gag gehört, wobei das NC auf Höhe des zweiten Zinkfingers mutiert ist, um die Sequenz des Proteins « Coat » des Bakteriophagen MS2 einzusetzen.

5. Lentivirales Partikel nach Anspruch 3, wobei:
- die Bindungsdomäne die Sequenz des Proteins « Coat » des Phagen PP7 ist,
- die Kapsidationssequenz der nicht-viralen RNS eine Haarnadelstruktursequenz von PP7 ist,
- das Nukleokapsidprotein das Protein von Nukleokapsid (NC) von HIV ist, das zum Polyprotein Gag gehört, wobei das NC auf Höhe des zweiten Zinkfingers mutiert ist, um die Sequenz des Proteins « Coat » des Bakteriophagen PP7 einzusetzen.

6. Zusammensetzung, die Partikel nach einem der Ansprüche 1 bis 5 umfasst.

7. Kit zur Herstellung von retroviralen Partikeln nach Anspruch 2, 4 oder 5, umfassend:
(i) ein Expressionsplasmid, das mindestens zwei verschiedene, nicht-virale Sequenzen von RNS kodiert, wobei jede RNS-Sequenz eine Sequenz von Interesse umfasst, für die vor oder nach dieser Sequenz von Interesse eine Kapsidationssequenz eingesetzt wird, oder alternativ ein erstes und ein zweites Expressionsplasmid, die jeweils eine verschiedene, nicht-virale RNS-Sequenz kodieren, die eine Sequenz von Interesse umfasst, und vor oder nach der Sequenz von Interesse eine Kapsidationssequenz eingesetzt wird,
(ii) ein Kapsidationsplasmid, das für ein chimäres Nukleokapsidprotein kodiert, das eine Bindungsdomäne umfasst, welche die Kapsidationssequenz erkennen lässt, und
(iii) ein Hüllplasmid, das für ein Hüllprotein kodiert.

8. Kit zur Herstellung von retroviralen Partikeln nach Anspruch 7, wobei die Sequenzen von Interesse verschieden sind und die Kapsidationssequenzen identisch sind.

9. Verfahren zur Fertigung eines Kits zur Herstellung von retroviralen Partikeln nach Anspruch 7, umfassend:
(i) Vorbereiten eines Expressionsplasmids, das mindestens zwei verschiedene, nicht-virale Sequenzen von RNS kodiert, wobei jede RNS-Sequenz eine Sequenz von Interesse umfasst, für die vor oder nach dieser Sequenz von Interesse eine Kapsidationssequenz eingesetzt wird, oder alternativ ein erstes und ein zweites Expressionsplasmid, die jeweils eine verschiedene, nicht-virale RNS-Sequenz kodieren, die eine Sequenz von Interesse umfasst, und in die vor oder nach der Sequenz von Interesse eine Kapsidationssequenz eingesetzt wird,
(ii) Vorbereiten eines Kapsidationsplasmids, das für ein chimäres Nukleokapsidprotein kodiert, das eine Bindungsdomäne umfasst, welche die Kapsidationssequenz erkennen lässt, und
(iii) Vorbereiten eines Hüllplasmids, das für ein Hüllprotein kodiert.

10. Verfahren zur Fertigung nach Anspruch 9 eines Kits zur Herstellung von retroviralen Partikeln nach Anspruch 8, wobei die Sequenzen von Interesse verschieden sind und die Kapsidationssequenzen identisch sind.

11. Verfahren zur Herstellung von retroviralen Partikeln nach Anspruch 2, 4 oder 5, umfassend einen Schritt zur Co-Transfektion von Zellen mit:
(i) einem Expressionsplasmid, das mindestens zwei verschiedene, nicht-virale Sequenzen von RNS kodiert, wobei jede RNS-Sequenz eine Sequenz von Interesse umfasst, für die vor oder nach dieser Sequenz von Interesse eine Kapsidationssequenz eingesetzt wird, oder alternativ ein erstes und ein zweites Expressionsplasmid, die jeweils eine verschiedene, nicht-virale RNS-Sequenz kodieren, die eine Sequenz von Interesse umfasst, und vor oder nach der Sequenz von Interesse eine Kapsidationssequenz eingesetzt wird,
(ii) einem Kapsidationsplasmid, das für ein chimäres Nukleokapsidprotein kodiert, das eine Bindungsdomäne umfasst, welche die Kapsidationssequenz erkennen lässt, und
(iii) einem Hüllplasmid, das für ein Hüllprotein kodiert,
und Auffangen des Überstands der die Partikel umfassenden transfizierten Zellen.

12. Verfahren zur Herstellung von retroviralen Partikeln nach Anspruch 11, wobei die Sequenzen von Interesse verschieden sind und die Kapsidationssequenzen identisch sind.

13. Verfahren zur Herstellung nach Anspruch 11 oder 12, wobei der Überstand geklärt wird.

14. Verfahren zur Herstellung nach Anspruch 13, wobei der Überstand darüber hinaus konzentriert und/oder gereinigt wird.

15. Verwendung eines Partikels nach einem der Ansprüche 1 bis 5 oder einer Zusammensetzung nach Anspruch 6, um Zellen *in vitro* oder *ex vivo zu* transduzieren.

16. Retrovirales Partikel nach einem der Ansprüche 1 bis 5 oder Zusammensetzung nach Anspruch 6 für seine bzw. ihre Verwendung in der Gentherapie.

## Claims

1. A retroviral particle comprising a protein coming from the Gag polyprotein, an envelope protein, optionally an integrase and at least two different encapsidated non-viral RNAs, the different encapsidated non-viral RNAs each comprising an RNA sequence of interest linked to an encapsidation sequence, each encapsidation sequence being recognized by a binding domain introduced into the protein coming from the Gag polyprotein.

2. A retroviral particle according to claim 1, comprising a nucleocapsid protein, an envelope protein, optionally an integrase and at least two different encapsidated non-viral RNAs, the different encapsidated non-viral RNAs each comprising an RNA sequence of interest linked to an encapsidation sequence, each encapsidation sequence being recognized by a binding domain introduced into the nucleocapsid protein.

3. A retroviral particle according to any one of claims 1 to 2, which is a lentiviral particle.

4. A lentiviral particle according to claim 3, in which
- the binding domain is the sequence of the Coat protein of the bacteriophage MS2,
- the encapsidation sequence of the non-viral RNAs is a stem-loop sequence of MS2,
- the nucleocapsid protein is the nucleocapsid protein (NC) of HIV belonging to the Gag polyprotein, which NC is mutated at the second zinc finger in order to insert the Coat protein sequence of the bacteriophage MS2.

5. A lentiviral particle according to claim 3, in which
- the binding domain is the sequence of the Coat protein of the bacteriophage PP7,
- the encapsidation sequence of the non-viral RNAs is a stem-loop sequence of PP7,
- the nucleocapsid protein is the nucleocapsid protein (NC) of HIV belonging to the Gag polyprotein, which NC is mutated at the second zinc finger in order to insert the Coat protein sequence of the bacteriophage PP7.

6. A composition comprising particles according to any one of claims 1 to 5.

7. A kit for producing retroviral particles according to claim 2, 4 or 5, comprising:
(i) an expression plasmid coding at least two different non-viral RNA sequences, each RNA sequence comprising a sequence of interest for which upstream or downstream of that sequence of interest is inserted an encapsidation sequence, or alternatively a first and a second expression plasmid each coding a different non-viral RNA sequence comprising a sequence of interest and upstream or downstream of said sequence of interest is inserted an encapsidation sequence,
(ii) an encapsidation plasmid coding for a chimeric nucleocapsid protein comprising a binding domain making it possible to recognize the encapsidation sequence, and,
(iii) an envelope plasmid coding for an envelope protein.

8. A kit for producing retroviral particles according to claim 7, in which the sequences of interest are different and the encapsidation sequences are identical.

9. A method of manufacturing a kit for producing retroviral particles according to claim 7, comprising:
(i) preparing an expression plasmid coding at least two different non-viral RNA sequences, each RNA sequence comprising a sequence of interest for which upstream or downstream of that sequence of interest is inserted an encapsidation sequence, or alternatively a first and a second expression plasmid each coding a different non-viral RNA sequence comprising a sequence of interest and in which upstream or downstream of the sequence of interest is inserted an encapsidation sequence,
(ii) preparing an encapsidation plasmid coding for a chimeric nucleocapsid protein comprising a binding domain making it possible to recognize the encapsidation sequence, and,
(iii) preparing an envelope plasmid coding for an envelope protein.

10. A manufacturing method according to claim 9, of a kit for producing retroviral particles according to claim 8, in which the sequences of interest are different and the encapsidation sequences are identical.

11. A method of manufacturing retroviral particles according to claim 2, 4 or 5, comprising a step of co-transfecting cells with:
(i) an expression plasmid coding at least two different non-viral RNA sequences, each RNA sequence comprising a sequence of interest for which upstream or downstream of that sequence of interest is inserted an encapsidation sequence, or alternatively a first and a second expression plasmid each coding a different non-viral RNA sequence comprising a sequence of interest and in which upstream or downstream of the sequence of interest is inserted an encapsidation sequence,
(ii) an encapsidation plasmid coding for a chimeric nucleocapsid protein comprising a binding domain making it possible to recognize the encapsidation sequence, and,
(iii) an envelope plasmid coding for an envelope protein,
and harvesting the supernatant of the transfected cells comprising the particles.

12. A method of manufacturing retroviral particles according to claim 11, in which the sequences of interest are different and the encapsidation sequences are identical.

13. A manufacturing method according to claim 11 or 12, in which the supernatant is clarified.

14. A manufacturing method according to claim 13, in which the supernatant is also concentrated and/or purified.

15. The use of a particle according any one of claims 1 to 5, or of a composition according to claim 6 for transducing cells in vitro or ex vivo.

16. A retroviral particle according to any one of claims 1 to 5 or a composition according to claim 6, for its use in gene therapy.
